# EUROPEAN PATENT APPLICATION

(11) **EP 2 572 730 A1**
(43) Date of publication of application: **27.03.2013**
(21) Application number: 11783655.1
(22) Date of filing: 20.05.2011
(51) Int. Cl.: A61K 45/00, A61K 31/7088, A61K 31/713, A61K 38/00, A61K 39/395, A61P 3/04, A61P 3/06

(54) **METHOD FOR PREVENTION OR TREATMENT OF METABOLIC SYNDROME**

(30) Priority: 20.05.2010 US 346843 P
(71) Applicant: Miyazaki, Toru, Tokyo 112-0003 (JP); Dainippon Sumitomo Pharma Co., Ltd., Osaka 541-8524 (JP)
(72) Inventor: Miyazaki, Toru, Tokyo 112-0003 (JP); Dainippon Sumitomo Pharma Co., Ltd., Osaka 541-8524 (JP)
(74) Representative: HOFFMANN EITLE
(86) International application number: PCT/JP2011/061653
(87) International publication number: WO 2011/145723

(57) **Abstract**

The present invention aims to provide a method for the prophylaxis or treatment of metabolic syndrome, which can discontinue the domino effect-like chain of diseases in metabolic syndrome in the upstream by suppressing infiltration of macrophage into the adipose tissues. The present invention provides a method for the prophylaxis or treatment of a metabolic syndrome, including a step of administering an AIM inhibitor to a subject.

## Description

### Technical Field

The present invention relates to a method for the prophylaxis or treatment of a metabolic syndrome, comprising administering an AIM inhibitor to a subject.

### Background Art

Conventionally, it is known that lifestyle-related diseases such as diabetes, hyperlipidemia, hypertension and obesity often accumulate in one person. While these diseases individually become a risk of arteriosclerosis, when accumulated, the risk becomes very high. Therefore, there are plural names that have been proposed to express the pathology of accumulation of these diseases.
At present, a disease concept including diabetes, hyperlipidemia, hypertension, obesity and insulin resistance as basic constituent factors, wherein these diseases often accumulate in one person and cause a high risk of arteriosclerotic diseases, is expressed by a standardized name of metabolic syndrome.

Generally, in metabolic syndrome, visceral fat type obesity, namely, obesity wherein neutral fats are intraperitoneally accumulated in excess, is the core pathology. The insulin resistance, which is developed due to obesity, sequentially causes hypertension, diabetes, hyperlipidemia and the like, and the accumulation and linkage thereof cause arteriosclerotic diseases. This phenomenon is compared to the domino effect, and the concept of "metabolic domino" has been proposed in recent years.
That is, a series of pathologies progress one directionally, like domino effect which is difficult to bring back once it starts to topple, and various diseases are developed chronically in a sustained manner over a long term.

The treatment of metabolic syndrome does not aim at mitigation of symptoms but aims at prevention of the onset of various diseases, particularly arteriosclerotic diseases, that may be developed thereafter.
As mentioned above, under the concept of metabolic domino, it is desirable to restrain progression at an upper stream, since the progression, once made, is difficult to bring back to the original state.

The studies conducted heretofore have clarified that, in metabolic syndrome, when excess neutral fats are accumulated in intraperitoneal adipocyte, abnormal secretion amount of adipocytokine from the adipocyte, increase of blood free fatty acid, and chronic inflammation of adipose tissue occur, as a result of which lifestyle-related diseases such as insulin resistance, diabetes, hyperlipidemia, hypertension and the like, as well as subsequent diseases are induced like a chain reaction.
Thus, it is considered preferable in the prophylaxis or treatment of metabolic syndrome to target upstream events such as adipocytokine secretion, blood free fatty acid and chronic inflammation of adipose tissue.

Among them, chronic inflammation of adipose tissue has been clarified to occur due to an increase of macrophage that infiltrated into the adipose tissues along with the obesity (see, for example, non-patent documents 1 and 2).
The activated state of macrophage includes 2 kinds: M1 and M2. M1 is also called classically activated macrophage, and shows increased production of inflammatory cytokines such as TNF-α, IL-6, IL-12 and the like, and inducible nitric oxide synthase (iNOS). M2 is also called alternatively activated macrophage, and shows decreased production of inflammatory cytokines and increased production of anti-inflammatory cytokines such as IL-10 and the like and arginase that inhibits iNOS activity.
It is known that macrophages that infiltrate into adipose tissue are mainly M2 in the case of non-obesity, and mainly M1 as obesity progresses (see, for example, non-patent document 3).

It has also been reported that, in macrophage-specific PPARγ-deficient mice, M2 is absent and high-fat diet induced obesity and insulin resistance are enhanced (non-patent document 4); inactivation of macrophage PPARγ leads to the deterioration of glucose tolerance and insulin resistance in skeletal muscle and the liver in normal diet nonobese mice (non-patent document 5); macrophage-specific Cap (Cbl-associated protein) deficient mice show decreased infiltration of macrophage in the adipose tissue, and attenuated expression of insulin resistance due to high-fat diet loading (non-patent document 6) and the like. Thus, M1 macrophages that infiltrated into adipose tissue are suggested to play an important role in the expression of insulin resistance.

The deep involvement of chronic inflammation of adipose tissue in the genesis of metabolic syndrome is consistent with a report that many of the patients with metabolic syndrome are high-sensitive CRP positive, which indicates the presence of mild inflammation, and the administration of salicylic acid and thiazolidine medicament having an anti-inflammatory action improves insulin resistance.
Moreover, in CCR2 (C-C chemokine receptor 2) knockout mice showing decreased infiltration of macrophage into adipose tissue, abnormal control of adipocytokine production associated with obesity is improved as compared to wild-type mice (non-patent document 7). Hence, macrophages that infiltrated into adipose tissue are considered to be also involved in the collapsed control of adipocytokine production.

From the above, it is considered that the progression of metabolic domino can be stopped at the most upstream site thereof such as collapsed control of adipocytokine production and chronic inflammation in adipose tissue, by targeting, in the prophylaxis or treatment of metabolic syndrome, inflammatory macrophages that infiltrated into adipose tissue.
However, the mechanism of induction of macrophages into adipose tissue along with the obesity has not been elucidated.
For example, reports have documented that macrophage induction efficiency decreases in monocyte chemotactic factor MCP-1 knockout mice and CCR2 knockout mice, but the efficiency varies depending on the reports. In addition, there is also a report stating that the macrophage infiltration is promoted in MCP-1 knockout mice.
Therefore, there is a room for discussion on the cause and effect between MCP-1 and CCR2, and macrophage induction, and at least they are not considered to be unambiguous (essential) and basic factors.
Naturally, a method of ceasing the progression of metabolic domino at the most upstream thereof by suppressing the basic cause of macrophage infiltration into adipose tissue has not been found.

In the meantime, the present inventors have found Apoptosis Inhibitor of Macrophage (AIM) from a tissue macrophage (see non-patent document 9). AIM is a soluble protein, and is a member of the scavenger receptor cysteine-rich (SRCR) superfamily. AIM was first found as an apoptosis inhibitor that protects macrophage from various apoptosis inducers (see non-patent document 9).
Since the structure of AIM, which has 3 SRCR domains, is similar to the extracellular domain of CD5, it is also called CD5L (CD5-like).

### [Document List]

### non-patent documents

non-patent document 1: Weisberg, S.P. et al., J. Clin. Invest., 2003, 112:1796-1808
non-patent document 2: Xu, H. et al., J. Clin. Invest., 2006, 116:115-124
non-patent document 3: Lumeng, C.N. et al., J. Clin. Invest., 2007, 117:175-184
non-patent document 4: Odegaard, J.L. et al., Nature, 2007, 447:1116-1121
non-patent document 5: Hevener, A.L. et al., J. Clin. Invest., 2007, 117:1658-1669
non-patent document 6: Lesniewski, L.A. et al., Nature Med., 2007, 13:455-462
non-patent document 7: Weisberg, S.P. et al., J. Clin. Invest., 2006, 116:115-124
non-patent document 8: Kanda, H. et al., J. Clin. Invest., 2006, 116:1494-1505
non-patent document 9: Miyazaki, T. et al., J. Exp. Med. 189, 413-422 (1999)
non-patent document 10: Arai, S. et al., Cell Metab. 1, 201-213 (2005)

### SUMMARY OF THE INVENTION

### Problems to be Solved by the Invention

The present invention aims to provide a method for the prophylaxis or treatment of metabolic syndrome, which can discontinue the domino effect-like chain of diseases in metabolic syndrome at the upstream by suppressing infiltration of macrophage into adipose tissue.

### Means of Solving the Problems

During further studies of AIM by the present inventors, we have found that macrophage incorporates lipid and produces AIM in an atherosclerotic lesion, and the produced AIM is also involved in the development of atherosclerosis since it supports the apoptosis resistance of macrophages in the lesion. Furthermore, we have produced AIM deficient mice, and confirmed that the AIM deficiency markedly decreases arteriosclerosis (Arai, S. et al., Cell Metab. 1, 201-213 (2005)).

In the meantime, the present inventors have found that AIM is also expressed in macrophages that infiltrated into obese adipose tissue as shown in the below-mentioned Reference Examples. In addition, we have found that differentiation of preadipocytes into mature adipocytes is suppressed in the presence of AIM; lysis of lipid droplet (lipolysis) is induced in mature adipocytes in the presence of AIM; the size of adipocytes tends to become small in the presence of AIM; and therefore, AIM itself is useful as an anti-obesity drug.
Moreover, we have also found that AIM produced in adipose tissue is bound to CD36 on the cell surface and incorporated into adipocytes by endocytosis; is bound to fatty acid synthase (FAS) in the cell and suppresses its activity, namely, the direct target molecule of AIM is FAS, and AIM induces suppression of differentiation of preadipocytes into mature adipocytes and lysis of lipid droplet through suppression of FAS activity.

However, the present inventors have now found a phenomenon which apparently seems opposite from the above-mentioned finding; namely, AIM is directly involved in the genesis of metabolic syndrome.
Specifically, we have confirmed by in vivo studies that blood concentration of AIM increases along with obesity; infiltration of macrophages is scarcely seen in adipose tissue of AIM knockout mice even after they have become obese; and inflammatory macrophages are infiltrated into adipose tissue by systemic administration of recombinant AIM to AIM knockout mice.
In addition, we have confirmed by in vitro studies that infiltration of inflammatory macrophages into adipose tissue is caused by migration of macrophages, which results from induction of lipid droplet fusion through suppression of FAS activity by AIM having increased blood concentration due to obesity.
Furthermore, we have found that glucose metabolism of AIM knockout mice is not degraded even when the mice become obese by taking a high-fat diet, and therefore, even if the mice become obese, inhibition of AIM can prevent subsequent chained onset and progression of a series of diseases of metabolic syndrome like domino effect, which resulted in the completion of the present invention.
Accordingly, the present invention relates to
[1] a method for the prophylaxis or treatment of metabolic syndrome or related diseases thereof, comprising a step of administering an AIM inhibitor to a subject;
[2] the method of the above-mentioned [1], wherein the aforementioned AIM inhibitor decreases stability of AIM in blood;
[3] the method of the above-mentioned [1], wherein the aforementioned AIM inhibitor inhibits binding between AIM and CD36;
[4] the method of the above-mentioned [1], wherein the aforementioned AIM inhibitor inhibits incorporation of AIM into a target cell;
[5] the method of the above-mentioned [1], wherein the aforementioned AIM inhibitor inhibits transfer of AIM from endosome to cytoplasm;
[6] the method of the above-mentioned [1], wherein the aforementioned AIM inhibitor inhibits binding of AIM to fatty acid synthase (FAS);
[7] the method of the above-mentioned [1], wherein the aforementioned AIM inhibitor suppresses an expression of AIM;
[8] the method of any one of the above-mentioned [1] - [7], wherein the aforementioned metabolic syndrome or related diseases thereof is at least one selected from the group consisting of metabolic syndrome, obesity, insulin resistance, diabetes, hyperlipidemia, hypertension, arteriosclerotic disease, hepatic disease, liver dysfunction, cerebrovascular disorder, ischemic heart disease, heart failure, dementia, cerebral apoplexy, neurosis, renal disease, secretion abnormality of adipocytokine, and abnormal amount of blood free fatty acid;
[9] the method of any one of the above-mentioned [1] - [8], wherein the aforementioned subject is a human;
[10] the method of any one of the above-mentioned [1] - [8], wherein the aforementioned subject is a non-human mammal or bird;
[11] the method of the above-mentioned [10], wherein the aforementioned subject is a dog or cat;
[12] an agent for the prophylaxis or treatment of metabolic syndrome or related diseases thereof, comprising at least one selected from the following group:
   an anti-AIM antibody;
   an anti-CD36 antibody;
   a double stranded nucleic acid having RNAi effect for an AIM gene;
   an antisense nucleic acid to an AIM gene;
   a ribozyme to an AIM gene;
   a protein binding to an anti-AIM antibody and not suppressing FAS activity;
   a protein binding to CD36 and not suppressing FAS activity;
   a protein binding to FAS and not suppressing FAS activity; and
   a soluble CD36;
[13] the therapeutic agent of the above-mentioned [12], wherein the aforementioned antibody is a monoclonal antibody, a polyclonal antibody, a recombinant antibody, a human antibody, a humanized antibody, a chimeric antibody, or an antibody fragment;
[14] the therapeutic agent of the above-mentioned [12] or [13], wherein the aforementioned protein binding to FAS and not suppressing FAS activity binds to at least one domain selected from the group consisting of DH, ER, TE and CC;
[15] the therapeutic agent of the above-mentioned [12] or [13], wherein the aforementioned protein binding to an anti-AIM antibody and not suppressing FAS activity is selected from the group consisting of an AIM fragment, an AIM variant or a fragment thereof, and an AIM chimeric protein or a fragment thereof;
[16] the therapeutic agent of the above-mentioned [12] or [13], wherein the aforementioned protein binding to CD36 and not suppressing FAS activity is selected from the group consisting of an AIM fragment, an AIM variant and an AIM chimeric protein;
[17] the therapeutic agent of the above-mentioned [12] or [13], wherein the aforementioned protein binding to FAS and not suppressing FAS activity is selected from the group consisting of an AIM fragment, an AIM analog, an AIM variant and an AIM chimeric protein;
[18] the therapeutic agent of any one of the above-mentioned [15] - [17], wherein the aforementioned AIM fragment is selected from fragments containing a functional domain of AIM protein and a conserved region thereof;
[19] the therapeutic agent of any one of the above-mentioned [12] - [18], wherein the aforementioned metabolic syndrome or related diseases thereof is at least one selected from the group consisting of metabolic syndrome, obesity, insulin resistance, diabetes, hyperlipidemia, hypertension, arteriosclerotic disease, hepatic disease, liver dysfunction, cerebrovascular disorder, ischemic heart disease, heart failure, dementia, cerebral apoplexy, neurosis, renal disease, secretion abnormality of adipocytokine, and abnormal amount of blood free fatty acid;
[20] the therapeutic agent of any one of the above-mentioned [12] - [19], wherein the aforementioned subject is a human;
[21] the therapeutic agent of any one of the above-mentioned [12] - [19], wherein the aforementioned subject is a non-human mammal or bird;
[22] the therapeutic agent of the above-mentioned [21], wherein the aforementioned subject is a dog or cat;
[23] a method for the prophylaxis or treatment of metabolic syndrome or related diseases thereof, comprising a step of administering the therapeutic agent of any one of the above-mentioned [12] - [22]; and
[24] a method of producing an AIM inhibitor, comprising
   a step of treating AIM with a protease to give an AIM fragment, and
   a step of purifying the aforementioned AIM fragment by an affinity column wherein an anti-AIM antibody binding to an AIM functional domain or conserved region is immobilized.

### Effect of the Invention

According to the method of the present invention, the chained onset of lethal disease group of metabolic syndrome can be stopped at the upstream thereof through suppression of infiltration of macrophage into adipose tissue in obese individual by inhibiting AIM.

### Brief Description of the Drawings

[Fig. 1] Fig. 1 shows the results of blood AIM concentration measurements of obese mice and normal mice.
[Fig. 2 ] Fig. 2 shows the results of detection, using anti-macrophage monoclonal antibody (F4/80) and the like, of macrophage infiltration in visceral fat tissues of obese AIM^{+/+} mice and obese AIM^{-/-} mice.
[Fig. 3] Fig. 3 shows the results of detection, using anti-macrophage monoclonal antibody (F4/80) and the like, of macrophage infiltration in visceral fat tissues at 3 weeks after systemic administration of rAIM to AIM^{-/-} mice.
[Fig. 4] Fig. 4 shows the measurement results of macrophage migration capability.
[Fig. 5] Fig. 5 are photographs showing the anatomy results of AIM^{+/+} mice and AIM^{-/-} mice after loading HFD for 12 weeks.
[Fig. 6] Fig. 6 shows the results of body weight and total fat amount of AIM^{+/+} mice and AIM^{-/-} mice after loading HFD for 12 weeks.
[Fig. 7] Fig. 7 shows the results of a glucose tolerance test performed on AIM^{+/+} mice and AIM^{-/-} mice before HFD loading.
[Fig. 8] Fig. 8 shows the results of a glucose tolerance test performed on AIM^{+/+} mice and AIM^{-/-} mice after HFD loading.
[Fig. 9] Fig. 9 shows the results of an insulin tolerance test performed on AIM^{+/+} mice and AIM^{-/-} mice before HFD loading.
[Fig. 10] Fig. 10 shows the results of an insulin tolerance test performed on AIM^{+/+} mice and AIM^{-/-} mice after HFD loading.
[Fig. 11] Fig. 11 shows the results of an insulin sensitivity test performed on AIM^{+/+} mice and AIM^{-/-} mice after HFD loading.
[Fig. 12] Fig. 12 shows the results of staining of sections produced from the visceral fat tissues of normal mice (mice without high-fat diet loading) and obese mice with anti-macrophage monoclonal antibody, anti-mouse AIM polyclonal antibody and anti-IL-6 antibody.
[Fig. 13] Fig. 13 shows the schedule of rAIM loading during culture of 3T3-L1 cell.
[Fig. 14] Fig. 14 shows the results of the cells stained with oil-red-O on Day 12 in the schedule shown in Fig. 13.
[Fig. 15] Fig. 15 shows the results of expression of adipocyte markers as measured by quantitative real-time PCR on day 12 in the schedule shown in Fig. 13.
[Fig. 16] Fig. 16A shows the results of mature adipocytes loaded with rAIM and stained with oil-red-O. Fig. 16B shows the size of lipid droplet and Fig. 16C shows the number of lipid droplet-containing cells per unit area.
[Fig. 17] Fig. 17 shows the measurement results obtained by loading mature adipocytes with rAIM, and measuring glycerol and free fatty acid in the culture supernatant.
[Fig. 18] Fig. 18 shows the measurement results obtained by loading mature adipocytes with rAIM, and measuring the expression of lipid droplet formation-related genes by quantitative real-time PCR.
[Fig. 19] Fig. 19 shows the results of HE staining of adipose tissue section of AIM^{+/+} mice and AIM^{-/-} mice after administration of HFD for 20 days.
[Fig. 20] Fig. 20A shows the results obtained by loading differentiated or undifferentiated 3T3-L1 cells with rAIM, and staining the cells for AIM, PPARγ2 and DAPI. Fig. 20B shows the measurement results obtained by classifying the cells according to the expression level of PPARγ2 based on the results of Fig. 20A, and counting the number of rAIM-containing cells per 100 of each cell. Fig. 20C shows the results obtained by loading 3T3-L1 cells with rAIM, and staining the cells for AIM and endosome, or AIM and lysosome.
[Fig. 21] Fig. 21 shows the results of electron microscopic observation of a sample same as that of Fig. 20 after labeling AIM with gold nanoparticles.
[Fig. 22] Fig. 22 shows the results obtained by treating 3T3-L1 cells with CD36 neutralizing antibody and examining the effect of rAIM for endocytosis.
[Fig. 23] Fig. 23 shows the results obtained by intravenously injecting rAIM to CD36^{+/+} mice and CD36^{-/-} mice, and staining sections prepared from the adipose tissue for AIM and macrophage.
[Fig. 24] Fig. 24 shows the results obtained by directly administering rAIM labeled with HA-tag by injection to adipose tissue of AIM^{-/-} mice, performing co-immunoprecipitation by anti-HA antibody using the adipose tissue, and detecting FAS in the precipitate by Western blotting.
[Fig. 25] Fig. 25 shows the results obtained by confirming the binding of rAIM labeled with HA-tag and FAS labeled with FLAG-tag by co-immunoprecipitation using an anti-Flag antibody or an anti-HA antibody in HEK 293T cells.
[Fig. 26] Fig. 26 shows the results obtained by labeling each domain of FAS with Flag-tag, expressing same in HEK 293T cell that stably expresses AIM-HA, and confirming the binding of FAS and AIM by co-immunoprecipitation using an anti-Flag antibody or an anti-HA antibody.
[Fig. 27] Fig. 27 shows the measurement results of FAS activity of 3T3-L1 cells treated in the presence or absence of rAIM (5 µg/mL), and in the presence of C75 (25 µM), for 6 days.
[Fig. 28] Fig. 28 shows the measurement results of FAS activity in adipose tissue of AIM^{+/+} mice and AIM^{-/-} mice.
[Fig. 29] Fig. 29 shows the measurement results of FAS activity in adipose tissue of AIM^{-/-} mice administered with rAIM or BSA 3 hours before by topical injection into adipose.
[Fig. 30] Fig. 30 shows amino acid sequences between human AIM and mouse AIM and the consensus.
[Fig. 31] Fig. 31 is a conceptual figure showing the structure of FAS.
[Fig. 32] Fig. 32 shows the measurement results of the changes in visceral fat amount and subcutaneous fat amount of AIM^{+/+} mouse and AIM^{-/-} mice administered with HFD for 12 weeks.
[Fig. 33] Fig. 33 shows the measurement results of the changes in the body weight of AIM^{-/-} mice for 5 weeks, during which the mice were administered with HFD along with rAIM or BSA twice a week.
[Fig. 34] Fig. 34 shows the measurement results of the changes in the visceral fat amount and subcutaneous fat amount of AIM^{-/-} mice for 5 weeks, during which the mice were administered with HFD along with rAIM or BSA twice a week.
[Fig. 35] Fig. 35 shows the measurement results of the mRNA levels of adipocyte marker and the like in the visceral fat of AIM^{-/-} mice after the experiments shown in Figs. 33 and 34.
[Fig. 36] Fig. 36 shows the detection results of AIM protein in the sera of dog, cat and mouse by Western blotting.
[Fig. 37] Fig. 37 shows the evaluation results of AIM suppressive activity of low-molecular-weight compounds obtained by screening, which is based on FSP27 expression.
[Fig. 38] Fig. 38 shows the evaluation results of AIM suppressive activity of low-molecular-weight compounds obtained by screening, which is based on FSP27 expression.
[Fig. 39] Fig. 39 shows the evaluation results of AIM neutralization activity of anti-AIM antibodies obtained by screening, which is based on FSP27 expression.
[Fig. 40] Fig. 40 shows the measurement results of blood AIM concentration of about 550 patients who underwent comprehensive medical examination.
[Fig. 41] Fig. 41 shows the measurement results of blood AIM concentration of blood donors (including foreigners) having BMI of 18 - 25 or not less than 35, who were selected at random.

### Description of Embodiments

The method for the prophylaxis or treatment of metabolic syndrome and related diseases of the present invention contains a step of administering an AIM inhibitor to a subject.
In the present specification, the metabolic syndrome is a concept showing a series of disease chain generally starting from visceral fat-type obesity (accumulation of visceral fat), then evoking insulin resistance via chronic inflammation of adipose tissue, secretion abnormality of adipocytokine from adipocyte, abnormal amount of blood free fatty acid and the like, thereafter inducing lifestyle-related diseases such as diabetes, hyperlipidemia, hypertension and the like, and finally developing various arteriosclerotic diseases. The downstream of the disease chain may include hepatic diseases, liver dysfunction, cerebrovascular disorder, ischemic heart diseases, heart failure, dementia, cerebral apoplexy, neurosis, renal diseases and the like.
In the present specification, the metabolic syndrome and related diseases include any diseases, symptoms and abnormalities based on various abnormalities produced in the mechanism of the onset or progression of metabolic syndrome, and in the process of the onset or progression of metabolic syndrome. For example, they include, but are not limited to, metabolic syndrome, obesity, insulin resistance, diabetes, hyperlipidemia, hypertension, arteriosclerotic diseases, hepatic diseases (including fatty liver, liver cancer), liver dysfunction, cerebrovascular disorders, ischemic heart diseases, heart failure, dementia, cerebral apoplexy, neurosis, renal diseases, abnormal secretion of adipocytokine, and abnormal amount of blood free fatty acid.

In the present specification, the prophylaxis or treatment of metabolic syndrome and related diseases is used in the broadest sense and means, for example, prevention, delay or improvement of insulin resistance; prevention, delay or improvement of the onset of metabolic syndrome and related diseases; mitigation of one or plural symptoms associated with metabolic syndrome and related diseases; improvement of the value of each item in the diagnosis criteria of metabolic syndrome and related diseases, and the like.
Examples of the items to be the diagnostic criteria of metabolic syndrome include waist diameter as an index of abdomen obesity, serum triglyceride value, HDL cholesterol value, blood pressure, fasting blood glucose level, glucose tolerance, insulin resistance, urine albumin content and the like.

In the present specification, an AIM inhibitor means a substance that inhibits the activity of AIM protein in the body by suppressing the function or expression of AIM protein. Examples thereof include, but are not limited to, low-molecular-weight compound, high-molecular-weight compound, peptide, protein, nucleic acid and the like.

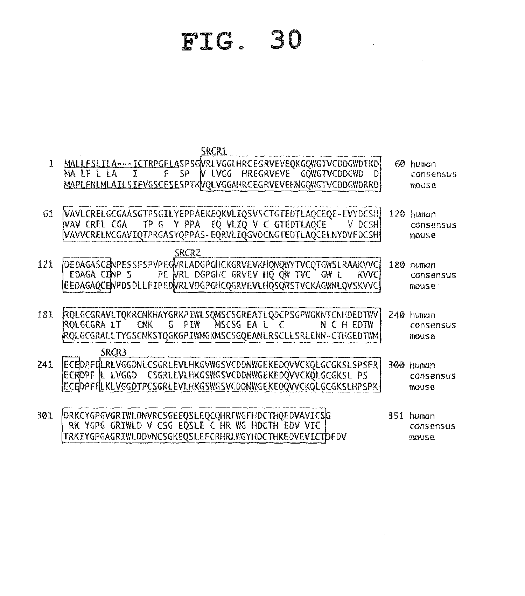

In the present specification, "AIM" is, as mentioned above, a soluble protein which is a member of the SRCR superfamily. As one example, amino acid sequences of human AIM and mouse AIM are shown in Fig. 30.

As shown in the below-mentioned Example 10, the blood AIM concentration is 5 - 20 µg/mL in most of the healthy individuals (those who are not under medical attention such as those who undergo a comprehensive medical examination). As shown in Example 11, moreover, subjects with BMI 35 or more show a significantly high blood AIM concentration as compared to those having BMI 18 - 25.

The AIM inhibitor of the present invention may inhibit any kind of AIM. For example, an inhibitor of AIM homologous protein in human, mammals other than human (e.g., mouse, rat, guinea pig, rabbit, dog, cat, swine, bovine, horse, monkey), birds and the like also corresponds to the AIM inhibitor of the present invention. Those of ordinary skill in the art can evaluate whether or not a protein is a homologous protein of AIM in other mammals or birds, from the high level of sequence similarity and functional analysis. For example, the homology of amino acid sequences between mouse AIM and human AIM is as high as about 80%. In addition, the consensus sequence in the SRCR domain (amino acid sequence conserved in other molecules having an SRCR domain such as CD5, CD6 and the like) is completely the same for mouse AIM and human AIM.
As examples, the amino acid sequences of AIM of human, chimpanzee, dog, mouse, and rat are shown in SEQ ID NOs: 1, 22, 23, 24 and 25, respectively.

In addition, the AIM inhibitor of the present invention may inhibit an analog or a variant of AIM. Examples of the analog and variant of AIM include a protein having an amino acid sequence of AIM wherein one to several amino acids have been deleted, substituted or added, and retaining the function of AIM. Those of ordinary skill in the art can determine whether a certain protein is an analog or a variant of AIM by the high level of sequence similarity or functional analysis.

In the present invention, the function of AIM to be inhibited by the AIM inhibitor means a function to infiltrate macrophages into an adipose tissue, and any function necessary for infiltration of macrophages into an adipose tissue and directly or indirectly exhibited by AIM. Examples of any function necessary for infiltration of macrophages into an adipose tissue and directly or indirectly exhibited by AIM include a function causing binding to CD36 on an adipocyte surface; a function causing incorporation into adipocytes by endocytosis; a function causing binding to FAS in adipocytes; a function causing suppression of enzyme activity of FAS; a function causing promotion of lipid droplet fusion; a function inducing macrophage migration by lipid droplet fusion, and the like.
By inhibiting any of these functions of AIM, infiltration of macrophage into an adipose tissue, which causes chronic inflammation of the adipose tissue and the whole body, can be prevented. As a result, even in obesity, insulin resistance is not induced, and chained onset of the diseases of metabolic syndrome can be stopped at the upstream.

(AIM inhibitor that suppresses function of AIM)
An AIM inhibitor that suppresses the function of AIM inhibits all or a part of the functions of AIM by directly or indirectly acting on AIM. Examples of the mechanism of suppressing AIM function include, but are not limited to, decreased stability of AIM in blood, inhibition of binding of AIM and CD36, inhibition of incorporation of AIM into a target cell, inhibition of incorporation of AIM into a target cell, inhibition of transfer of AIM from endosome into cytoplasm, inhibition of binding of AIM and FAS, and the like.

When an AIM inhibitor that decreases stability of AIM in blood is administered, AIM is degraded in a short time without serving the function thereof.

When an AIM inhibitor that inhibits binding of AIM and CD36 is administered, incorporation of AIM into a target cell by endocytosis can be inhibited, whereby the function of AIM can be suppressed.
As a substance inhibiting binding of AIM and CD36, any substance that inhibits binding between proteins can be used and, for example, an anti-AIM antibody and an anti-CD36 antibody can be mentioned. As shown in the below-mentioned Reference Examples, administration of an anti-CD36 antibody can suppress incorporation of AIM into a target cell. As the anti-AIM antibody, one recognizing the binding site of AIM to CD36 as an epitope is preferable. As the anti-CD36 antibody, one recognizing the binding site of CD36 to AIM is preferable.

The "antibody" in the present specification also includes an antibody fragment, and can be a monoclonal antibody, a polyclonal antibody, a recombinant antibody, a human antibody, a humanized antibody, a chimeric antibody, a single-chain antibody, a Fab fragment, a F(ab')₂ antibody, scFv, a bispecific antibody, a synthetic antibody and the like.
These antibodies can be produced according to a method known to those of ordinary skill in the art. For example, an anti-AIM monoclonal antibody can be obtained by isolating antibody-producing cells from a non-human mammal immunized with AIM, fusing this with myeloma cell and the like to produce hybridoma, and purifying the antibody produced by the hybridoma. In addition, the polyclonal antibody can be obtained from a serum of an animal immunized with AIM, and the like.
AIM to be used here for immunization may be full-length or a fragment, and can be appropriately determined by those of ordinary skill in the art. When it is a fragment, it preferably contains the binding site to CD36.

In addition, when a non-human monoclonal antibody that efficiently inhibits binding of AIM to CD36 can be obtained, it can also be produced by a genetic recombinant method. For example, total RNA is prepared by a standard method from a hybridoma producing the anti-AIM monoclonal antibody, mRNA encoding the anti-AIM antibody is prepared using a commercially available kit, and cDNA is synthesized using the reverse transcriptase, whereby a DNA encoding the anti-AIM antibody can be obtained. An expression vector containing the DNA is transfected into a suitable host cell and cultivated under suitable conditions to express the anti-AIM antibody.

In addition, a DNA encoding a CDR region of an anti-AIM antibody can also be obtained by a PCR method using the above-mentioned cDNA as a template. Utilizing a DNA encoding the CDR region, a human antibody and a humanized antibody can be produced by a genetic recombinant method according to a conventional method. For example, a DNA encoding a CDR region derived from a non-human antibody and a DNA designed to link to a framework region of a human antibody are synthesized by PCR method, and further linked to a DNA encoding a human antibody constant region, whereby a DNA encoding a human antibody can be obtained.
Such DNA is inserted into an expression vector (e.g., plasmid, retrovirus, adenovirus, adeno-associated virus (AAV), plant virus such as cauliflower mosaic virus, tobacco mosaic virus and the like, cosmid, YAC, EBV-derived episome) according to a known method (a method of utilizing restriction enzyme etc.), and the expression vector is transfected into a suitable host cell to give a transformant. The expression vector can further contain a promoter to regulate expression of an antibody gene, a replication origin, a selection marker gene and the like. The promoter and replication origin can be appropriately selected according to the kind of the host cell and the vector.
Then, the transformant is cultivated under suitable conditions to express a human antibody of the anti-AIM antibody.
As the host cell, for example, eukaryotic cells such as mammalian cell (CHO cell, COS cell, myeloma cell, HeLa cell, Vero cell etc.), insect cell, plant cell, fungi cell (Saccharomyces, Aspergillus etc.), and prokaryotic cells such as Escherichia coli (E. Coli), Bacillus subtilis and the like can be used.

The expressed antibody can be isolated and purified by an appropriate combination of known methods (e.g., affinity column using protein A and the like, other chromatography column, filter, ultrafiltration, salting out, dialysis etc.).
When the anti-AIM antibody of the present invention is a low-molecule antibody such as Fab fragment, F(ab')₂ antibody, scFv and the like, it can also be expressed using a DNA encoding a low-molecule antibody and according to the above-mentioned method. In addition, the antibody can also be produced by a treatment with an enzyme such as papain, pepsin and the like.

As an AIM inhibitor that inhibits binding of AIM and CD36, a protein that binds to an anti-AIM antibody and dose not suppress FAS activity can also be used. Such protein binds competitively with AIM to CD36, and inhibits suppression of FAS activity due to the binding of AIM to CD36.
Examples of the protein that binds to an anti-AIM antibody and dose not suppress FAS activity include AIM fragment, AIM variant or a fragment thereof, and AIM chimeric protein or a fragment thereof.
While the AIM fragment is not particularly limited as long as it consists of a partial peptide of AIM, for example, a fragment of 5 - 150 amino acids containing AIM functional domain or conserved region can be mentioned.
As the AIM variant, a protein having an amino acid sequence of AIM, wherein 1 - 10 amino acids have been deleted, substituted or added, and not suppressing FAS activity can be mentioned.
The AIM chimeric protein means a chimeric protein of a part of a human AIM protein and an AIM protein derived from other animal (e.g., mouse).
The protein that binds to an anti-AIM antibody and dose not suppress FAS activity also includes a fragment of AIM variant or AIM chimeric protein.
The protein that binds to an anti-AIM antibody and dose not suppress FAS activity can be expressed by obtaining, by a conventional method, a DNA encoding the protein and allowing the DNA to express the protein by a genetic recombinant method. To increase the affinity to CD36 and stability in blood, the amino acid sequence can be altered and modified as appropriate. Where necessary, the protein may also be expressed as a fusion protein with other protein and peptide.
Moreover, AIM fragment, a fragment of AIM variant and a fragment of AIM chimeric protein can also be obtained by producing each full-length protein and treating the protein with a protease.

Furthermore, as an AIM inhibitor that inhibits binding of AIM and CD36, a protein that binds to CD36 and dose not suppress FAS activity can also be used. Such protein binds competitively with AIM to CD36, and inhibits suppression of FAS activity by binding of AIM to CD36.
Examples of the protein that binds to CD36 and has dose not suppress FAS activity include AIM fragment, AIM variant or a fragment thereof, and AIM chimeric protein or a fragment thereof.

When an AIM inhibitor that inhibits incorporation of AIM into a target cell is administered, the function of AIM to bind to FAS in the target cell and suppress an enzyme activity thereof can be inhibited.

In addition, an AIM inhibitor that inhibits transfer of AIM from the endosome into the cytoplasm also inhibits the function of AIM to bind to FAS in the target cell and suppress an enzyme activity thereof.

When an AIM inhibitor that inhibits binding of AIM and FAS is administered, the action of AIM on FAS can be directly inhibited and, for example, a protein that binds to FAS and dose not suppress FAS activity can be mentioned. Such protein binds competitively with AIM to FAS, and inhibits suppression of FAS activity by binding of AIM to FAS.
As such protein, a protein that binds to at least one domain of FAS selected from the group consisting of DH, ER, TE and CC is preferable.
Examples of the protein that binds to FAS and dose not suppress FAS activity include AIM fragment, AIM variant or a fragment thereof, and AIM chimeric protein or a fragment thereof.

The AIM inhibitor that suppresses the function of AIM, which is explained above, can be easily selected from the candidate compounds by a screening method utilizing the facts that AIM suppresses differentiation of preadipocytes into mature adipocytes, induces lipid droplet fusion in adipocytes, reduses the size of adipocytes and the like.
For example, an AIM inhibitor can be easily selected by a screening method including
(i) a step of cultivating preadipocyte under conditions for differentiation into adipocyte, and adding only AIM or AIM and a candidate compound to a medium,
(ii) a step of evaluating differentiation of the aforementioned preadipocytes into adipocytes, and
(iii) a step of selecting a candidate compound that increases differentiation induction efficiency when added together with AIM, as compared to the addition of AIM alone.
Step (ii) can be performed using, for example, production of lipid droplet in adipocytes, and expression of adipocyte marker, preadipocyte marker and/or mesenchymal stem cell marker as indices.

In addition, the AIM inhibitor can also be easily selected by a screening method including
(a) a step of culturing adipocytes, and adding only AIM or AIM and a candidate compound to a medium,
(b) a step of evaluating lipid droplet fusion in the aforementioned adipocytes, and
(c) a step of selecting a candidate compound that decreases lipid droplet fusion efficiency when added together with AIM, as compared to the addition of AIM alone.
Step (b) can be performed using, for example, the amount of glycerol or free fatty acid in the culture supernatant of adipocyte, expression of lipid droplet formation-related gene and the like as indices.

In addition, the AIM inhibitor can be easily selected by a screening method including
(1) a step of culturing adipocytes, and adding only AIM or AIM and a candidate compound to a medium,
(2) a step of evaluating the size of the aforementioned adipocytes, and
(3) a step of selecting a candidate compound that increases the size of adipocytes when added together with AIM, as compared to the addition of AIM alone.
Step (2) can be performed by, for example, hematoxylineosin staining of cells.

In addition, the AIM inhibitor includes
a step of cultivating adipocytes or preadipocytes in a medium including AIM in the presence or absence of a candidate compound, and
a step of evaluating AIM uptake into the aforementioned cells after the aforementioned culture.
The AIM uptake into a cell can be detected by, for example, after the cultivation step of the cell, fixing the cell, imparting permeability, adding an anti-AIM antibody labeled to permit detection, and incubating the cell.
When the uptake into the cell decreases in the presence of a candidate compound, the candidate compound can be evaluated to inhibit the function of AIM by inhibiting incorporation of AIM into the cell.

Moreover, the AIM inhibitor includes
a step of cultivating adipocytes or preadipocytes in a medium including AIM in the presence or absence of a candidate compound, and
a step of evaluating the binding of AIM and FAS in the aforementioned cells.
The binding of AIM and FAS can be confirmed by, for example, preparing cell lysates, immunoprecipitation using an anti-AIM antibody, and immunoblotting the immunoprecipitate using an anti-FAS antibody.
When the binding of AIM and FAS decreases in the presence of a candidate compound, the candidate compound can be evaluated to inhibit the function of AIM by inhibiting binding of AIM and FAS.

As a low-molecular-weight compound that inhibits AIM function, the following compounds can be mentioned. 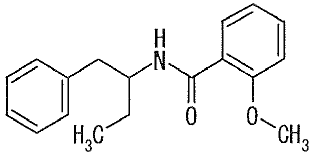 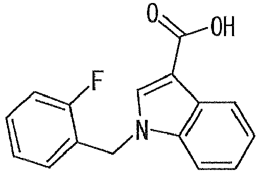 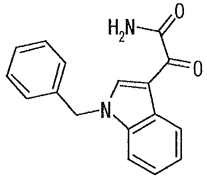 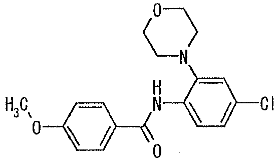 As shown in the below-mentioned Examples, these compounds were confirmed to significantly suppress remarkable decrease of the expression of lipid droplet formation-related gene FSP27 mRNA originally induced by the addition of AIM, and inhibit the function of AIM.

As a molecule that inhibits the function of AIM, the neutralizing antibodies produced in the below-mentioned Example 9 (clones 11, 12 and 17) can also be mentioned. These neutralizing antibodies were also confirmed to significantly suppress remarkable decrease of the expression of lipid droplet formation-related gene FSP27 mRNA originally induced by the addition of AIM.

### (AIM inhibitor that suppresses an expression of AIM)

The AIM inhibitor that suppresses an expression of AIM suppresses all or a part of the functions of AIM by decreasing the level of AIM expression in macrophage.
Examples of the AIM inhibitor that suppresses an expression of AIM include a double stranded nucleic acid which has an RNAi effect for an AIM gene, antisense nucleic acid, ribozyme, and nucleic acid encoding them.

The RNAi effect is a mechanism for suppressing sequence-specific gene expression induced by a double stranded nucleic acid. It is a highly safe method since it has extremely high target specificity and utilizes a gene expression suppressive mechanism originally present in vivo.
As the double stranded nucleic acid having an RNAi effect, for example, siRNA can be mentioned. When siRNA is used for a mammalian cell, it is a double stranded RNA generally having about 19 - 30 bases, preferably about 21 bases - 25 bases. As an AIM inhibitor of the present invention, moreover, a longer double stranded RNA which can be cleaved by an enzyme (Dicer) into siRNA can also be used.
A double stranded nucleic acid having an RNAi effect generally has one base sequence complementary to a part of the target nucleic acid, and the other sequence complementary thereto. The double stranded nucleic acid having an RNAi effect generally has two protruding bases (overhang) at each 3'-terminal, but may also be a blunt end type with no overhang. For example, 25 bases blunt end RNA is advantageous in that it minimizes activation of an interferon responsive gene, prevents a sense strand-derived off-target effect, and shows extremely high stability in serum, and is also suitable for use in vivo.
The double stranded nucleic acid having an RNAi effect can be designed based on the base sequence of the target gene and according to a known method. In addition, the double stranded nucleic acid having an RNAi effect may also be a double stranded RNA, a DNA-RNA chimera-type double stranded nucleic acid, an artificial nucleic acid or a nucleic acid with various modifications.

An antisense nucleic acid has a base sequence complementary to the target gene (basically mRNA as a transcription product), and is generally a single strand nucleic acid having 10 base length - 100 base length, preferably 15 base length - 30 base length. When an antisense nucleic acid is introduced into a cell, gene expression is inhibited by hybridizing with a target gene. The antisense nucleic acid may not be completely complementary to the target gene as long as it affords an inhibitory effect on the target gene expression. The antisense nucleic acid can be appropriately designed by those of ordinary skill in the art by using known software and the like. The antisense nucleic acid may be any of DNA, RNA and DNA-RNA chimera, or may be modified.

Ribozyme is a nucleic acid molecule that catalytically hydrolyzes a target RNA, and is constituted with an antisense region having a sequence complementary to the target RNA and a catalyst center region in charge of cleavage reaction. Ribozyme can be appropriately designed by those of ordinary skill in the art according to a known method. Ribozyme is generally an RNA molecule, and can also be used as a DNA-RNA chimera-type molecule.

A nucleic acid encoding any of the aforementioned double stranded nucleic acid which has an RNAi effect, antisense nucleic acid and ribozyme can also be used as the AIM inhibitor of the present invention. When a vector containing such nucleic acid is introduced into macrophage, a double stranded nucleic acid which has an intracellular RNAi effect, an antisense nucleic acid and ribozyme, are expressed, each exhibiting an effect to suppress an expression of AIM.
As the nucleic acid for encoding double stranded nucleic acid having an RNAi effect, a DNA encoding each of the double strands may be used, or a DNA encoding a single strand nucleic acid, wherein double stranded nucleic acids are connected via a loop, may also be used. In the latter case, a single strand RNA is obtained by intracellular transcription and its complementary part is hybridized in a molecule to form a hairpin type structure. This RNA is called an shRNA (short hairpin RNA). When shRNA is transferred into the cytoplasm, the loop is cleaved by an enzyme (Dicer) to form a double stranded RNA, which exhibits an RNAi effect.

In the method for the prophylaxis or treatment of metabolic syndrome in the present invention, the aforementioned AIM inhibitor is administered to a subject.
The "subject" in the present specification includes any living organisms which can be affected by metabolic syndrome because of the infiltration of macrophage into adipose tissue caused by AIM, and is not particularly limited. For example, human, mammals other than human (e.g., mouse, rat, rabbit, dog, cat, swine, bovine, horse, monkey), birds and the like can be mentioned.
For example, mouse AIM and human AIM show high amino acid sequence homology of about 80%. In addition, mouse AIM and human AIM have completely identical consensus sequences in the SRCR domain (amino acid sequence also conserved in other molecules having SRCR domain such as CD5, CD6 and the like), the method of the present invention is considered to be also effective for other animals.
In addition, pets such as dog, cat, and the like have a problem of metabolic syndrome in recent years, which is caused by too much high-fat feed and lack of activity. Therefore, the method of the present invention is also particularly useful for these animals.

The method of the present invention is preferably used for a subject having a blood AIM concentration higher than the normal level. As shown in the Examples described below, the blood AIM concentration increases with obesity.
On the other hand, the present inventors have heretofore found that, when the blood AIM concentration is low - medium concentration, AIM suppresses differentiation of preadipocyte into adipocyte in adipose tissues, and promotes lipid droplet fusion.
From such findings, it can be said that, when the blood AIM concentration is low - medium, AIM shows an anti-obesity effect in adipose tissue, but at high concentrations, introduces macrophage into adipose tissue and evokes inflammation of the adipose tissue.
Therefore, when an AIM inhibitor is used as an agent for the prophylaxis or treatment of metabolic syndrome, the blood AIM concentration in the subject is preferably higher than the normal level.

In the present invention, the AIM inhibitor can be orally or parenterally administered systemically or topically. For example, intravenous injection such as drip and the like, intramuscular injection, intraperitoneal injection, subcutaneous injection, suppository, enema, oral enteric agent and the like can be selected, and an appropriate administration method can be selected according to the age and symptom of the patient.

In addition, the AIM inhibitor of the present invention may contain a pharmaceutically acceptable carrier such as preservative, stabilizer and the like. The pharmaceutically acceptable carrier means a material that can be administered to a subject together with the AIM inhibitor (active ingredient). The pharmaceutically acceptable carrier is not particularly limited as long as it is a pharmacologically and pharmaceutically acceptable. Examples thereof include, but are not limited to, water, saline, phosphate buffer, dextrose, glycerol, pharmaceutically acceptable organic solvents such as ethanol etc., collagen, polyvinyl alcohol, polyvinylpyrrolidone, carboxyvinyl polymer, sodium carboxymethyl cellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerol, glycerol, propylene glycol, petrolatum, paraffin, stearylalcohol, stearic acid, human serum albumin, mannitol, sorbitol, lactose, surfactant, excipient, flavoring agent, preservative, stabilizer, buffering agent, suspension, isotonicity agent, binder, disintegrant, lubricant, flowability promoter, corrigent and the like.

The AIM inhibitor of the present invention can be formulated into a general form of medical preparation. The medical preparation is appropriately prepared using the above-mentioned carriers. The form of medical preparation is not particularly limited, and is appropriately selected and used according to the treatment object. Representative examples thereof include tablet, pill, powder, liquid, suspension, emulsion, granule, capsule, suppository, injection (liquid, suspension, emulsion) and the like. These preparations can be produced by a method generally employed.

When the AIM inhibitor of the present invention contains a nucleic acid, it can be formulated by enclosing the nucleic acid in a carrier such as liposome, polymer micell, cationic carrier and the like. In addition, a nucleic acid carrier such as protamine may also be utilized. It is also preferable to bind an antibody and the like to the carrier to target the affected part. Furthermore, it is also possible to enhance blood retention by binding cholesterol and the like to the nucleic acid. Moreover, when the AIM inhibitor of the present invention contains a nucleic acid encoding siRNA and the like, which is expressed in the cell after administration, the nucleic acid can also be inserted into a virus vector such as retrovirus, adenovirus, Hemagglutinating Virus of Japan and the like, or a non-virus vector such as liposome and the like and intracellularly introduced.

In addition, the amount of the active ingredient contained in the AIM inhibitor of the present invention can be appropriately determined by those of ordinary skill in the art according to the kind of the active ingredient. For example, in the case of an antibody such as anti-AIM antibody and the like, the dose may be, but is not limited to, 0.025 - 50 mg/kg, preferably 0.1 - 50 mg/kg, more preferably 0.1 - 25 mg/kg, further preferably 0.1 - 10 mg/kg or 0.1 - 3 mg/kg.
Moreover, the AIM inhibitor of the present invention is also preferably administered in combination with other medicaments having the same action and effect as the AIM inhibitor, such as anti-arteriosclerotic drug, lipid lowering drug, FAS inhibitor and the like. Examples of the medicament that can be administered in combination with the AIM inhibitor include, but are not limited to, at least one anti-arteriosclerotic drug selected from the group consisting of Probucol, Fibrates, Statins, Aspirin and Coxibs, and at least one FAS inhibitor selected from the group consisting of C75, C93, Cerulenin, PHS11A, 2-octanoic acid, thiolactomycin, and a compound represented by the following formula (1) wherein R¹ is a hydrogen atom or a methyl group or a pharmacologically acceptable salt thereof. 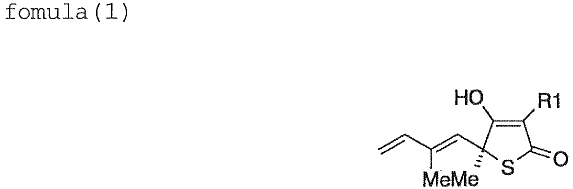 The combined administration is not limited as to the administration period or dose of each of the two or more medicaments administered as long as it shows an additional or synergistic effect, and they may be administered simultaneously or separately.

As mentioned above, AIM protein has heretofore been recognized to cause suppression of differentiation to adipocyte and lipid droplet fusion (lipolysis), and itself show an anti-obesity effect. As shown in the below-mentioned Examples, the AIM protein has now been comfirmed to induce infiltration of macrophage into adipose tissue. In addition, it has been shown that when the AIM protein is knocked out, glucose metabolism is not aggravated even in visceral fat-type obesity.
According to the method for the prophylaxis or treatment of metabolic syndrome of the present invention, therefore, administration of an AIM inhibitor prevents infiltration of M1 macrophage into an adipose tissue to prevent chronic inflammation of adipose tissue and the whole body, as a result of which chain of diseases that progress like domino effect can be stopped at the upstream thereof. As a result, metabolic syndrome can be basically prevented and treated.

The present invention also encompasses a production method of an AIM inhibitor.
The production method of an AIM inhibitor includes a step of treating AIM with a protease to give a partial peptide of AIM, and a step of purifying said partial peptide by an affinity column wherein an anti-AIM antibody that binds to a functional domain or conserved region of AIM is fixed. Examples

The present invention is specifically explained in the following based on the Examples, which are not to be construed as limitative.

### Example 1. Increase of blood AIM concentration due to obesity

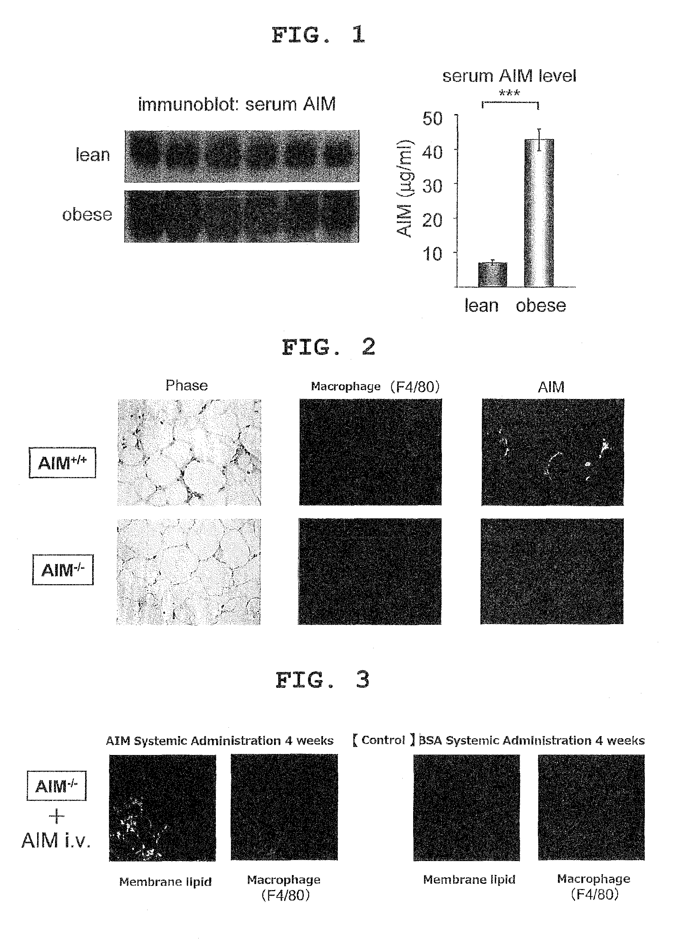

Serum AIM concentration of obese mice prepared by administering a high-fat diet (HFD, fat calorie: 60%) to C57BL. 6 (B6) mice for 20 weeks, and normal mice was measured. The results are shown in Fig. 1.
The serum AIM concentration of the obese mice (obese) was 4 times or more high as compared to that of the normal mice (lean).

### Example 2. Induction of infiltration of macrophage into adipose tissue by AIM

Visceral fat tissues were collected from AIM^{+/+} mice and AIM^{-/-} mice fed with HFD to become fat, and paraffin sections produced from the adipose tissues were stained with anti-macrophage monoclonal antibody (F4/80), anti-mouse AIM polyclonal antibody (SA-1) and anti-IL-6 antibody (MP520F3, R & D systems).
The results are shown in Fig. 2. As shown in Fig. 2, M1 macrophage infiltrated into the adipose tissue in the normal mice (AIM^{+/+}) expressing AIM, but infiltration of macrophage into adipose tissue was scarcely found in the AIM knockout mice (AIM^{-/-}).
As mentioned above, AIM has a suppressive function of macrophage apoptosis. Therefore, when macrophage is not detected in an adipose tissue of an AIM knockout mouse, there is a possibility that macrophage infiltrated into the adipose tissue and apoptosis occurred. To confirm this possibility, apoptosis of macrophage in the adipose tissues of obese AIM knockout mice and obese normal mice was measured. As a result, the level thereof was not different (data not shown).
Thus, it was confirmed that macrophage was not detected in the adipose tissue because macrophage did not infiltrate into the adipose tissue.

Then, rAIM was systemically administered to the AIM knockout mice by intravenous injection (50 µg/body/injection). After the administration was continued for 3 weeks, a section produced from the visceral fat tissue was stained with an anti-macrophage monoclonal antibody (F4/80).
The results are shown in Fig. 3. As shown in the Figure, it was observed that systemic administration of rAIM to an AIM knockout mice result in the infiltration of macrophages into adipose tissue.
From the results mentioned above, it is shown that AIM induces infiltration of macrophages into adipose tissue, and therefore, inhibition of AIM can suppress infiltration of macrophages into adipose tissue even in visceral fat-type obesity.

### Example 3. Elucidation of mechanism of macrophage migration

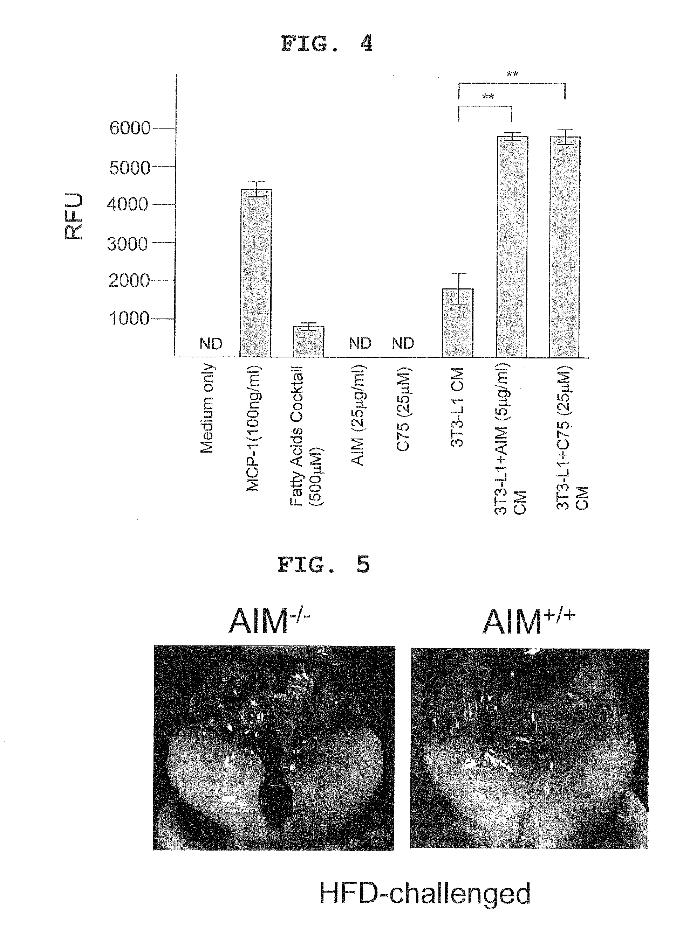

The migration capability of mouse macrophage cell line RAW264.8 (1 x 10⁵/well, 24 hr) was analyzed.
CytoSelect™ 96-Well Cell Migration Assay (CELL BIOLABS, Inc., 5 mm, Fluorometric format) was used. The results are shown in Fig. 4. Values obtained by subtracting the background (medium only) from respective RFU values are shown. Fatty Acids Cocktail consists of Myristoleic acid, Palmitic acid, Oleic acid and Linoleic acid.
Cell supernatant (3T3-L1 CM) obtained by culturing, for 6 days, mature 3T3-L1 adipocytes on day 8 from differentiation induction, supernatant obtained by co-culture with rAIM (3T3-L1+AIM CM), and supernatant obtained by co-culture with C75 (3T3-L1+C75 CM) were used. CM: conditioned medium, ND: not detected. **: p<0.01
As shown in the below-mentioned Reference Examples, AIM induces lipid droplet fusion in mature adipocytes. As a result, free fatty acid and glycerol derived from lipid droplet are released in the culture supernatant.
Combined with the results of the macrophage migration experiment, it is strongly suggested that AIM having an increased blood concentration along with the obesity induces lipid droplet fusion in adipose tissue, which induces infiltration of macrophages into adipose tissue.

### Example 4. Suppression of decrease in glucose metabolism of obese AIM knockout mice

Wild-type mice and AIM knockout mice were loaded with HFD for 12 weeks, and glucose metabolism was measured and an insulin sensitivity test was performed, before (Lean) and after (Obese) the loading, by a glucose tolerance test and an insulin tolerance test.
In the glucose tolerance test, the blood glucose level was measured every 30 min for 120 min after the glucose loading (3 g/kg body; intraperitoneal injection), and in the insulin tolerance test, the blood glucose level was measured every 30 min for 120 min after the insulin loading (0.75 U/kg body; intraperitoneal injection).
The insulin sensitivity test was performed by administering insulin to AIM^{+/+} mice and AIM^{-/-} mice after loading with HFD (0.75 U/kg body;intravenous injection), collecting tissues (white adipose tissue, gastrocnemius muscle and liver) 2 hr later, purifying protein, and measuring phosphorylated AKT and phosphorylated GSK3β by Western blotting.
In addition, the body weight and total amount of fat of each mouse were measured after HFD loading.

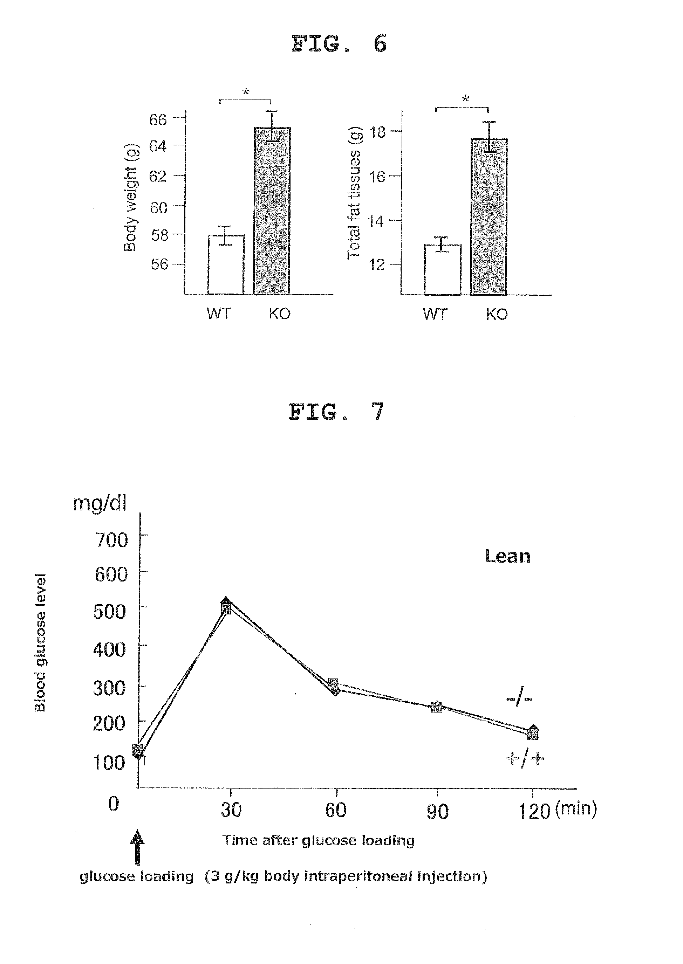

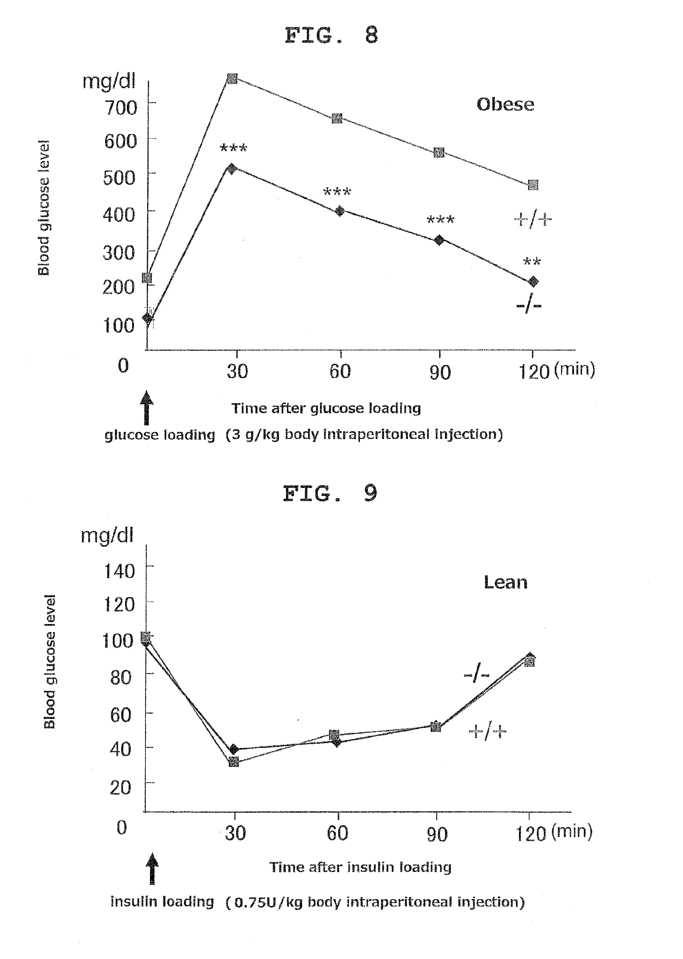

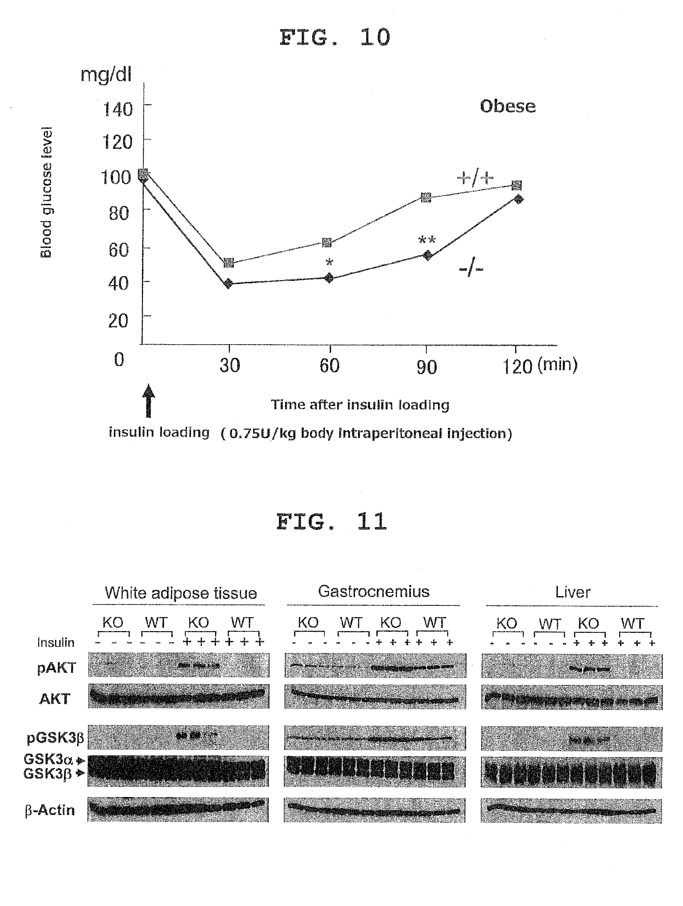

Fig. 5 shows the results of anatomy after loading, Fig. 6 shows the measurement results of body weight and total amount of fat, Figs. 7 - 10 show the measurement results of glucose metabolism, and Fig. 11 shows the results of the insulin sensitivity test.
As shown in Figs. 5 and 6, HFD loading caused visceral-type obesity in both of the AIM knockout mice (AIM^{-/-}) and normal mice (AIM^{+/+}). Increase in the body weight and adipose tissue amount was more remarkable in the AIM knockout mice.
However, as shown in Figs. 7 - 10, glucose metabolism markedly decreased in the obese normal mice, but the glucose metabolism did not decrease in the obese AIM knockout mice.
Furthermore, as shown in Fig. 11, phosphorylated AKT and phosphorylated GSK3β were expressed in the AIM^{-/-} mice after insulin administration, and it was confirmed that signal transduction via an insulin receptor functioned, that is, the insulin sensitivity was normal. On the other hand, phosphorylated AKT and phosphorylated GSK3β were not detected in the wild-type mice, thus confirming the presence of insulin resistance.
In addition, it was also confirmed by a hyperinsulinemic-euglycemic clamp test that the AIM^{-/-} mice had normal insulin sensitivity (data not shown).
The above-mentioned results support that, in obese AIM knockout mice, macrophage does not infiltrate into adipose tissue, and therefore, inflammation reaction of adipose tissue and the whole body is suppressed and the insulin sensitivity does not decrease.
The isles of Langerhans in the pancreas was separated from the obese AIM knockout mice or normal mice, and production and secretion of insulin was measured by glucose loading. As a result, no difference was found between the both. Therefore, it was confirmed that the difference in the glucose tolerance is not based on insulin production or secretion but on difference in insulin sensitivity.

### Reference Example 1. Expression of AIM in macrophage in adipose tissue

Visceral fat tissues were collected from lean mice (lean) obtained by feeding a general feed to wild-type B6 mice and obese mice (obese) obtained by feeding a high-fat diet (HFD, fat calorie 60%) for 20 weeks, and paraffin sections produced from the adipose tissues were double stained with anti-macrophage monoclonal antibody (F4/80), anti-mouse AIM polyclonal antibody (SA-1) and anti-IL-6 antibody (MP520F3, R & D systems).

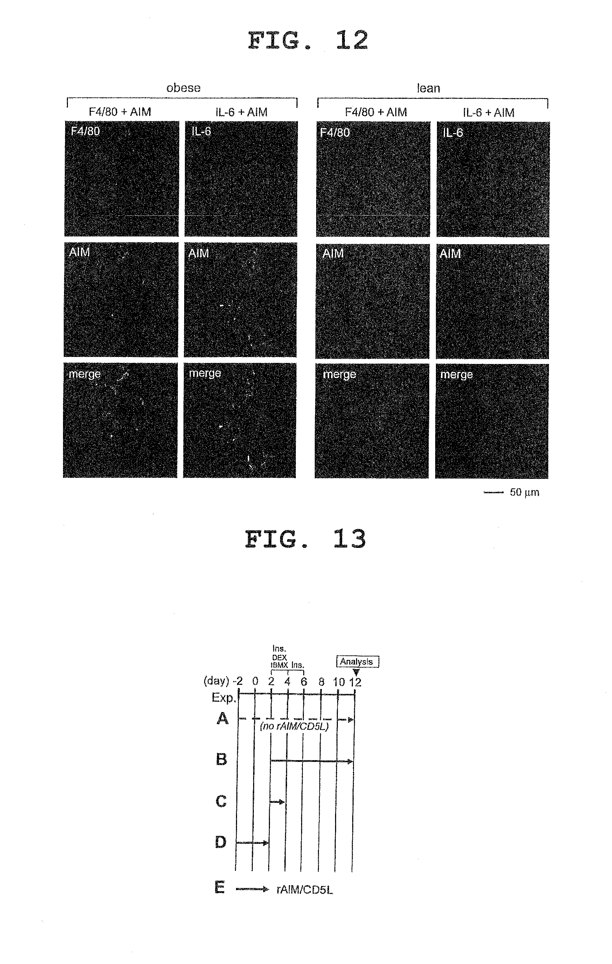

Fig. 12 shows one example of the results of observation of the stained section with a fluorescence microscope.
The macrophage in adipose tissue of the obese mice, which was stained with an anti-macrophage antibody, was also stained with an AIM specific antibody (obese left lane). Since AIM positive macrophage was also positive for IL-6 (obese right lane), it was considered an inflammatory macrophage (M1).
On the other hand, the macrophage in the adipose tissue collected from the lean mice was negative for both AIM and IL-6.
To confirm that AIM was not expressed in adipocyte, adipose tissue derived from obese mice (epididymal adipose tissue was used as representative visceral tissue) was fractionated after collagenase treatment, and expression of AIM was examined by RT-PCR. However, AIM expression was not found in purified adipocyte. Furthermore, 3T3-L1 cells were treated with insulin, DEX and IBMX, and AIM expression was examined. As a result, expression was not found.
From the above-mentioned results, it was confirmed that macrophage that infiltered into adipose tissue strongly expressed AIM.

### Reference Example 2. Suppressive function on differentiation of preadipocyte into adipocyte by AIM

### [Evaluation by lipid droplet formation]

To examine the function of AIM produced by macrophage that infiltrated into adipose tissue on the surrounding adipocytes, an experiment was performed to load AIM in the culture process for differentiation of 3T3-L1 preadipocytes into mature adipocytes.
As shown in Fig. 13, 3T3-L1 cells were cultured in four schedules A - D. In (A), AIM was not loaded, in (B), AIM was loaded for 10 days from the start of the differentiation induction stimulation (day 2-day 12), in (C), AIM was loaded only in the initial stage of differentiation induction stimulation (day 2-day 4), and in (D), AIM was loaded only in the clonal expansion period before differentiation induction (day(-2)-day 2).

As AIM, recombinant AIM protein (rAIM) of mouse was used. rAIM was prepared by culturing human-derived HEK293T cells transfected with a vector expressing mouse AIM (pCAGGS-mAIM-HA plasmid) in a serum-free medium (FreeStyle™ 293 Expression Medium; Invitrogen). rAIM was purified from the culture supernatant thereof using an anti-HA antibody column. In other Reference Examples shown below, AIM used was a similar rAIM protein. AIM was loaded by adding AIM to a culture medium at a concentration of 5 µg/ml.

In addition, differentiation of 3T3-L1 cells was induced as shown in Fig. 13 by first proliferating 3T3-L1 cells by cultivating for 4 days (day(-2)-day 2), after which cultivating in a culture medium containing insulin, dexamethasone (DEX) and isobutylmethylxanthine (IBMX) for 2 days (day 2-day 4) to start differentiation induction stimulation, and further cultivating in a culture medium containing insulin for 2 days (day 4-day 6).

After the differentiation induction stimulation period (day 2 - day 6), the cells were continuously cultured in a culture medium free of such differentiation induction stimulation factors. Then, the cells on day 10 day from the start of the differentiation induction stimulation (day 12) were stained with oil-red-O, and the state of differentiation of 3T3-L1 cells into mature adipocytes was observed.

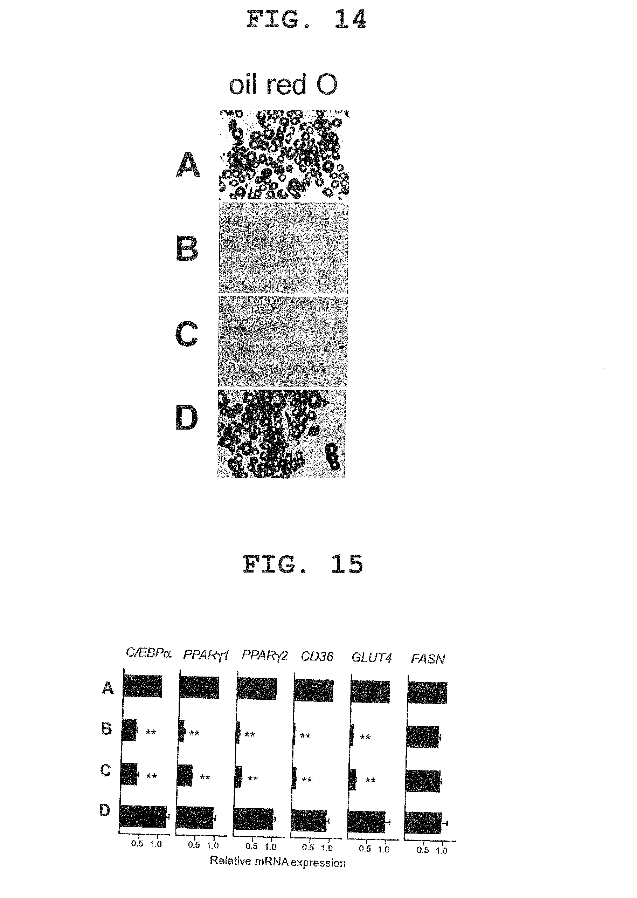

Fig. 14 shows microscopic photographs of the respective stained cells of 4 schedules A - D.

As shown in Fig. 14, when AIM was loaded only in the initial stage (day 2 - day 4) of differentiation induction stimulation in schedule C, cells having lipid droplet were scarcely observed, and differentiation of 3T3-L1 cells was nearly completely suppressed. In other words, AIM suppressed differentiation of 3T3-L1 cells in the presence of the aforementioned differentiation inducer.

Even when AIM was removed from a culture medium containing a differentiation inducer and AIM, by using a purification column after preparation of the medium, the ability to differentiate the 3T3-L1 cells by said differentiation inducer was not lost (data not shown). Therefore, no substantive chemical interaction was considered to be present between the differentiation inducer and AIM.

In addition, when AIM was loaded for 10 days (day 2-day 12) from the start of the differentiation induction stimulation in schedule B, cells having lipid droplet were scarcely observed, and the differentiation of 3T3-L1 cells was nearly completely suppressed. Furthermore, such differentiation suppressive effect of AIM was confirmed to depend on the concentration of AIM (data not shown). When the concentration of AIM loaded on the 3T3-L1 cells was decreased to 1 µg/ml and 0.1 µg/ml, the differentiation suppressive effect of AIM also decreased.

Moreover, the number of dead cells did not increase due to AIM loading. Therefore, it was considered that suppression of differentiation by AIM did not induce cell death but was caused by decrease in the number of 3T3-L1 cells that differentiated into mature adipocytes.

On the other hand, when AIM was loaded only before differentiation induction (day(-2) - day 2) in schedule D, it was confirmed that almost all cells had lipid droplet, and almost all 3T3-L1 cells differentiated into mature adipocytes, as in the case of no loading of AIM in schedule A. That is, even when AIM was loaded only before differentiation induction, differentiation of 3T3-L1 cells into mature adipocytes was not suppressed. However, when AIM was continuously loaded from before differentiation induction through differentiation induction period and thereafter (day(-2) - day 12), differentiation of 3T3-L1 cells into adipocytes was completely suppressed (data not shown).

When human rAIM was used for AIM loading, differentiation of mouse-derived 3T3-L1 cells was similarly suppressed. This shows that the function of AIM is interchangeable between human and mouse.

### [Evaluation by expression of adipocyte markers]

The cells cultured in schedules A - D shown in Fig. 13 were recovered on day 10 from the start of the differentiation induction stimulation (day 12), and expression of adipocyte markers (C/EBPα, PPARγ1, PPARγ2, CD36, Glut4) at mRNA level was measured using 7500 Fast Real-Time PCR system (Invitrogen; CA, USA) by ΔΔC_{T} method. The primers used are shown in Table 1.

**Table 1**

| | |
|---|---|
| f-PPARγ1 | CAAGAATACCAAAGTGCGATCAA (SEQ ID NO:2) |
| r-PPARγ1 | GAGCTGGGTCTTTTCAGAATAATAAG (SEQ ID NO:3) |
| f-PPARγ2 | CCAGAGCATGGTGCCTTCGCT (SEQ ID NO:4) |
| r-PPARγ2 | CAGCAACCATTGGGTCAGCTC (SEQ ID NO:5) |
| f-C/EBPα | AGCAACGAGTACCGGGTACG (SEQ ID NO:6) |
| r-C/EBPα | TGTTTGGCTTTATCTCGGCTC (SEQ ID NO:7) |
| f-CD36 | TTGTACCTATACTGTGGCTAAATGAGA (SEQ ID NO:8) |
| r-CD36 | CTTGTGTTTTGAACATTTCTGCTT (SEQ ID NO:9) |
| f-Glut4 | GACGGACACTCCATCTGTTG (SEQ ID NO:10) |
| r-Glut4 | GCCACGATGGAGACATAGC (SEQ ID NO:11) |

The results are shown in Fig. 15. It was shown that adipocyte markers were expressed and preadipocytes were differentiated into adipocytes in schedules A and D. On the other hand, expression of adipocyte markers were suppressed in schedules B and D in which AIM was administered simultaneously with differentiation induction stimulation, and suppression of differentiation into adipocyte was suggested. The results are consistent with schedules A and D where lipid droplet was formed and schedules B and D where it was not formed.

As mentioned above, AIM suppresses differentiation of preadipocytes into adipocytes. In addition, the level of differentiation can be examined easily by observation of lipid droplet formation or detection of adipocyte marker, preadipocyte marker or mesenchymal stem cell marker.
Therefore, as a result of lipid droplet formation or detection of marker, when the differentiation induction efficiency increases when AIM and a candidate compound are added together as compared to the addition of AIM alone, said candidate compound can be evaluated to be a compound that suppresses the function of AIM, and when the differentiation induction efficiency decreases, said candidate compound can be evaluated to promote the function of AIM.

### Reference Example 3. Function of limpid droplets fusion of adipocyte by AIM

### [Evaluation by oil-red-O]

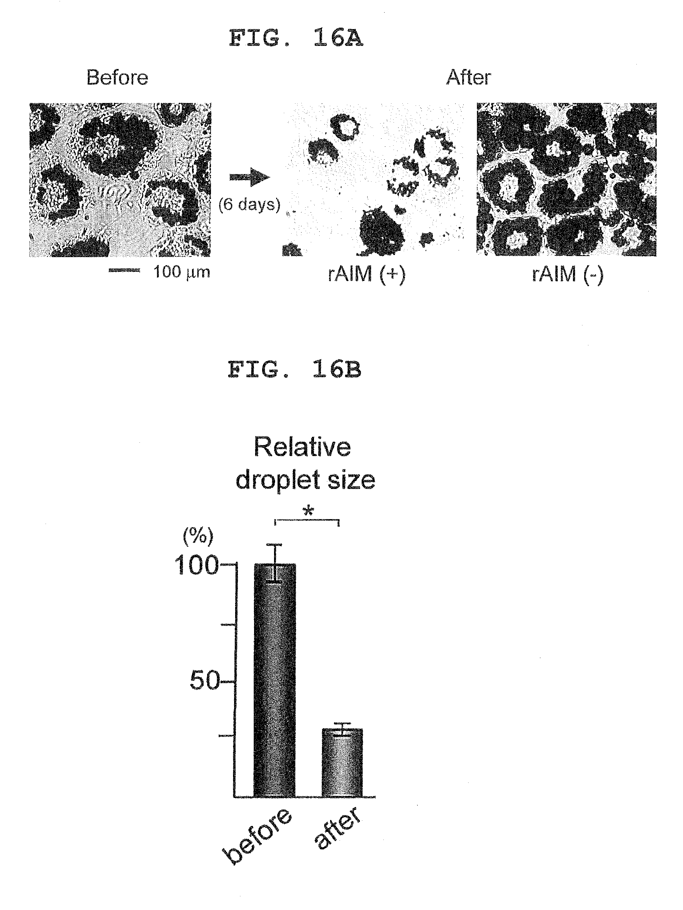

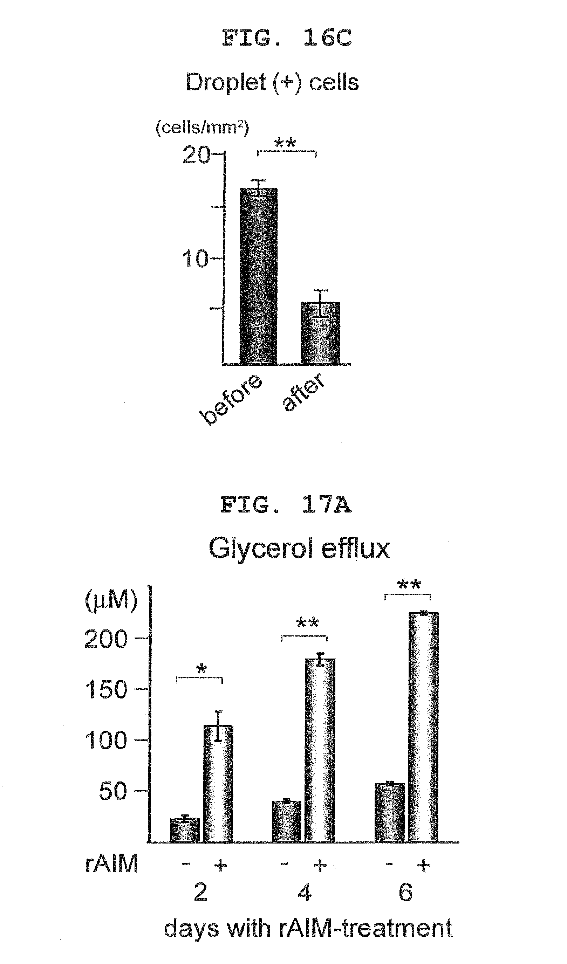

The differentiated 3T3-L1 cells were loaded with rAIM (5 µg/ml) according to the schedule shown in Fig. 13, E. The oil-red-O staining was performed before and after rAIM loading. Representative photograph is shown in Fig. 16A. The lipid droplet in the cell markedly decreased after rAIM loading followed by 6 days of culture (rAIM(+)).
The relative size of lipid droplet (Relative droplet size) was determined from an average diameter of 50 lipid droplets. The error bar shows standard error. The number of cells containing lipid droplets per unit area was measured in 5 different fields and the average was determined. Each result is shown in Figs. 16B, C.
The relative size of lipid droplets and the number of lipid droplet-containing cells significantly decreased.
The results suggest that rAIM induces lipid droplet fusion, and glycerol and free fatty acids contained in the lipid droplets were released in the supernatant.

### [Evaluation by amount of glycerol or free fatty acids in culture supernatant]

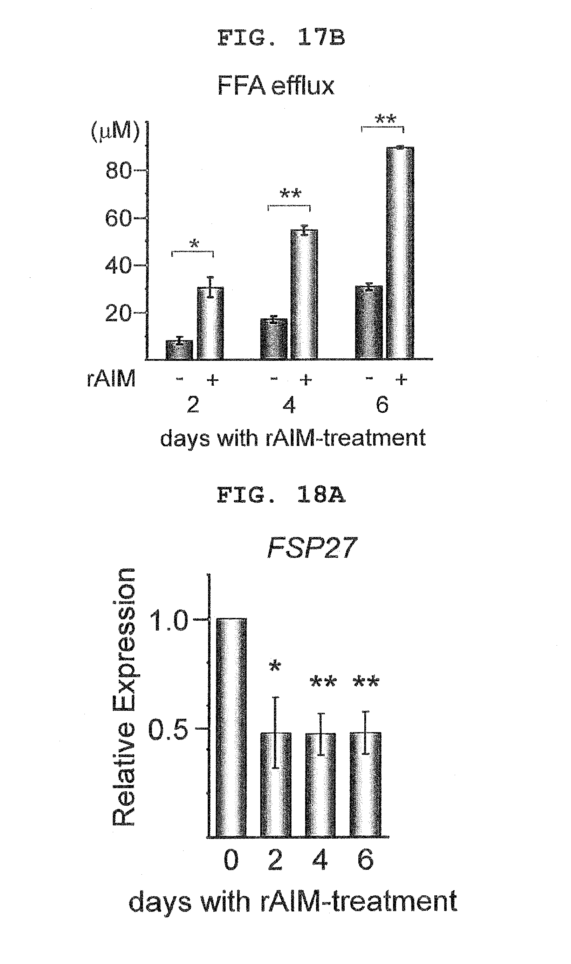

rAIM was loaded (5 µg/ml) on 3T3-L1 adipocytes, the amount of glycerol or free fatty acid released in the culture supernatant was measured on day 2, day 4, and day 6. After rAIM loading, the cells were washed twice with PBS, and incubated in a serum-free medium (FreeStyle™ 293 Expression Medium; Invitrogen) for 5 hr. The buffer was recovered, and the measurement was performed using a glycerol assay kit and a fatty acid assay kit (both Bio Vision Inc.) and according to the manual.
The results are shown in Fig. 17. When rAIM was added before cultivation, the amounts of glycerol and free fatty acids in the culture supernatant significantly increased.
The results suggest that rAIM induced lipid droplet fusion, and glycerol and free fatty acids contained in the lipid droplet were released in the supernatant.

### [Evaluation by expression of lipid droplet formation-related genes]

rAIM was loaded (5 µg/ml) on 3T3-L1 adipocytes, mRNA of lipid droplet formation-related genes (FSP27, Perilipin, and Adipophilin) was measured on day 2, day 4, and day 6 using 7500 Fast Real-Time PCR system (Invitrogen; CA, USA) and by the ΔΔC_{T} method (each n=3).
Similarly, mRNA of PREF-1 of preadipocyte marker was also measured. The measurement value was standardized with GAPDH. The primers used are shown in Table 2.

**Table 2**

| | |
|---|---|
| f-Fsp27 | CTGGAGGAAGATGGCACAATCGTG (SEQ ID NO:12) |
| r-Fsp27 | CAGCCAATAAAGTCCTGAGGGTTCA (SEQ ID NO:13) |
| f-Perilipin | TGCTGGATGGAGACCTC (SEQ ID NO:14) |
| r-Perilipin | ACCGGCTCCATGCTCCA (SEQ ID NO:15) |
| f-Adipophilin | AAGCATCGGCTACGACGACAC (SEQ ID NO:16) |
| r-Adipophilin | GGACAGTCTGGCATGTAGTCTGGA (SEQ ID NO:17) |
| f-GAPDH | AACTTTGGCATTGTGGAAGG (SEQ ID NO:18) |
| r-GAPDH | GGATGCAGGGATGATGTTCT (SEQ ID NO:19) |
| f-PREF1 | CGGGAAATTCTGCGAAATAG (SEQ ID NO:20) |
| r-PREF1 | TGTGCAGGAGCATTCGTACT (SEQ ID NO:21) |

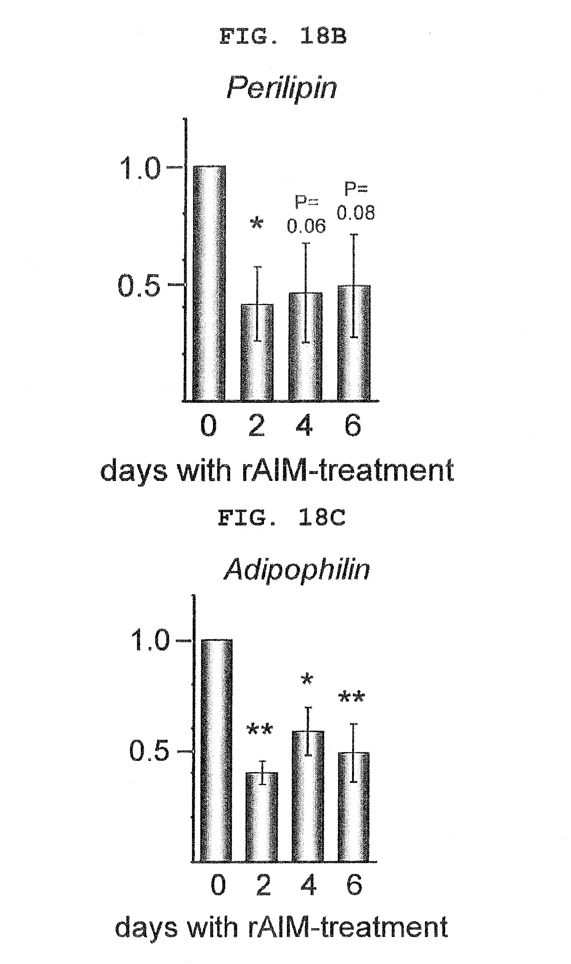

The results are shown in Fig. 18. Relative expression level was determined with that on day 0 as 1.0. The error bar shows standard error.
The expression of lipid droplet formation-related genes had already decreased markedly on day 2 after rAIM treatment. On the other hand, the expression of preadipocyte markers did not increase during rAIM treatment. This suggests that AIM does not cause dedifferentiation of mature adipocyte.

As mentioned above, AIM fuses lipid droplet in mature adipocyte. The level of lipid droplet fusion can be examined easily by observation with a microscope, measurement of glycerol or free fatty acid level of the culture supernatant, measurement of the expression of lipid droplet formation-related gene and the like.
Therefore, as a result of detection of glycerol and the like in the culture supernatant, when the lipid droplet fusion efficiency decreases when AIM and a candidate compound are added together as compared to the addition of AIM alone, said candidate compound can be evaluated to be a compound that suppresses the function of AIM, and when the lipid droplet fusion efficiency increases, said candidate compound can be evaluated to promote the function of AIM.

### Reference Example 4. Function on reducing the size of adipocytes by AIM

### [Evaluation by HE staining]

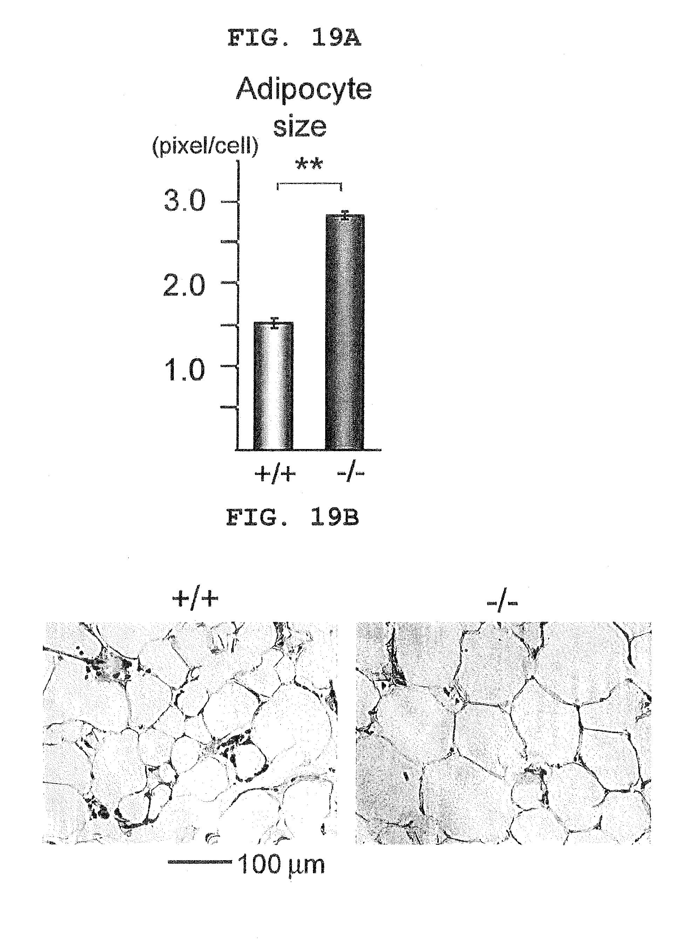

AIM^{+/+} mice (+/+) and AIM^{-/-} mice (-/-) (see non-patent document 1) were fed with a high-fat diet (HFD) for 20 days, and a section of adipose tissue was stained with HE. In various fields of a microscope, the distance among 50 adipocytes was measured, and mean±standard error was determined. The results are shown in Fig. 19A, and representative photograph is shown in Fig. 19B. The adipocytes of AIM^{-/-} mice were significantly large as compared to AIM^{+/+} mice.

As mentioned above, AIM reduces the size of adipocytes. The level of reduction can be examined easily by a microscope.
Therefore, as a result of measurement of the size, when the cell size increases when AIM and a candidate compound are added together as compared to the addition of AIM alone, said candidate compound can be evaluated to be a compound that suppresses the function of AIM, and when the cell size decreases, said candidate compound can be evaluated to promote the function of AIM.

### Reference Example 5. AIM uptake into adipocyte via CD36 on cell surface

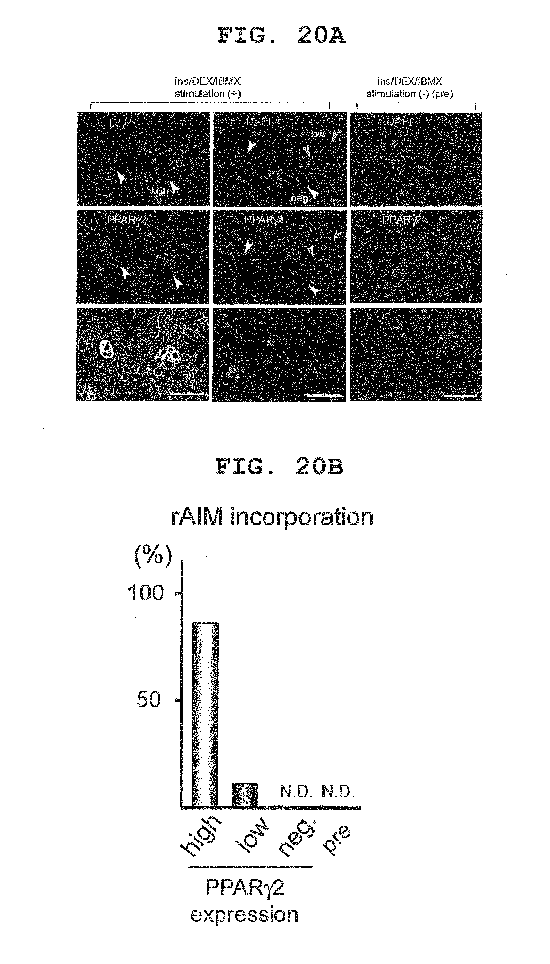

Differentiated 3T3-L1 cells (ins/DEX/IBMX stimulation (+)) and undifferentiated 3T3-L1 cells (ins/DEX/IBMX stimulation (-)) were incubated with rAIM (5 µg/ml) for 3 hr, and stained with AIM, PPARγ2, and DAPI. For staining with PPARγ2, a rabbit anti-PPARγ2 polyclonal antibody (Abcam plc) was used. The results are shown in Fig. 20A.
The upper panel shows the results of nucleus stained with AIM and DAPI, the middle panel shows the results of staining with AIM and PPARγ2, and the lower panel shows the results of phase contrast image of staining with AIM, PPARγ2 and DAPI. It was observed that AIM was dispersed in the cytoplasm of adipocyte.
As is clear from the lower panel, the cells highly expressing PPARγ2 contains many lipid droplets. The cells highly expressing PPARγ2 are sufficiently differentiated mature adipocytes. The right lane indicated as "pre" shows undifferentiated 3T3-L1 preadipocyte without differentiation induction stimulation.
Fig. 20B shows the number of cells containing rAIM per 100 cells which was obtained by classifying the cells according to the expression level of PPARγ2. As shown in the Figure, it was found that mature adipocytes highly expressing PPARγ2 showed efficient rAIM uptake, and the cells showing low expression of PPARγ2, which were not sufficiently differentiated, showed markedly low rAIM uptake efficiency. The preadipocytes (pre) free of differentiation induction stimulation showed no rAIM uptake.

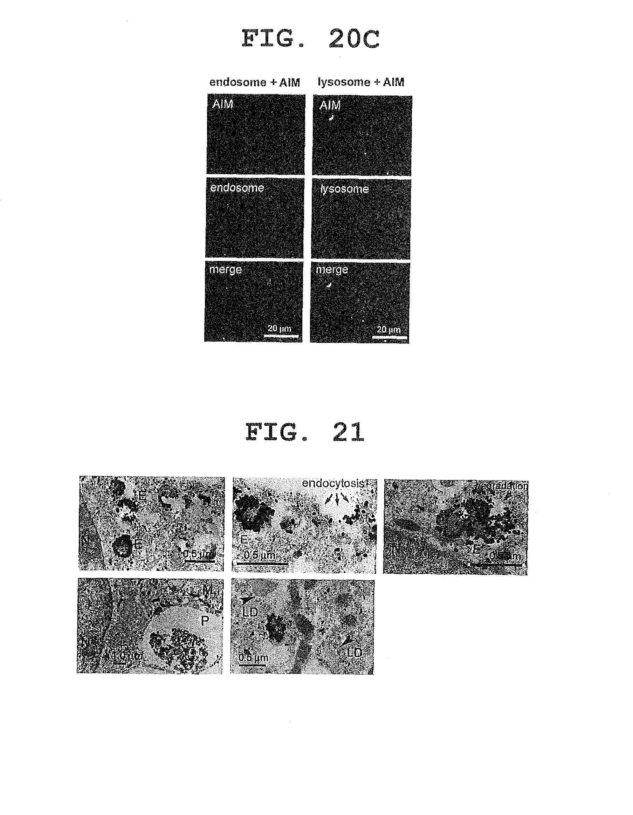

In addition, 3T3-L1 adipocytes were treated with rAIM for 3 hr, stained with AIM and endosome (FM 1-43FX(Invitrogen Corporation) was used), AIM and lysosome (Lyso Tracker Red DND-99(Invitrogen Corporation) was used), and observed under a confocal microscope. The results are shown in Fig. 20C.
rAIM incorporated into adipocyte was co-localized with endosome, and co-localization with lysosome was not observed.

Using the same sample, AIM was labeled with gold nanoparticles, and observed under an electron microscopy. Specifically, the cells pre-treated with normal goat serum for 30 min were fixed with para-formaldehyde, and incubated overnight with SA-1 rabbit anti-AIM polyclonal antibody (1:600 dilution). Then, they were reacted with goat anti-rabbit IgG covalently bound with 1 nm gold nanoparticles (1:200; Nanoprobes, Inc.). The cells were silver enhanced using HQ silver (Nanoprobes, Inc.), osmificated, dehydrated, and directly embedded in Epon (Nisshin EM Corporation.). An extremely thin section was prepared, stained with uranyl acetate and lead citrate, and observed with an electron microscope (H-7100; Hitachi Inc.).
The results are shown in Fig. 21. E shows endosome, P shows phagosome or phagolysosome, M shows mitochondria, N shows nucleus, and LD shows lipid droplet.
rAIM labeled with gold nanoparticles has an endosome-like structure, and particularly accumulated near the limiting membrane thereof (upper left panel). In the cellular membrane, endocytosis of rAIM was observed (upper center panel). Some particles containing rAIM were found near the nucleus, and considered to be late endosome (upper right panel). The late endosome is denatured, along with which rAIM is released in the cytoplasm.
AIM was not detected in phagosome and phagolysosome having a large diameter and an irregular shape, mitochondria and lipid droplet (lower panel).
Putting the above results together, it is suggested that AIM is incorporated into adipocyte by endocytosis, and exhibits function in the cell.

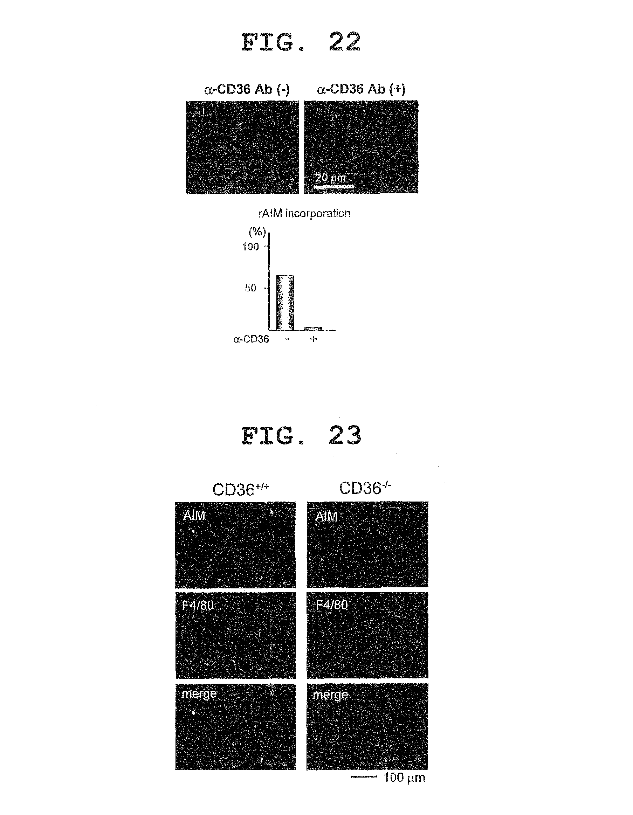

Then, the cell surface molecule involved in AIM uptake was specified. Attention was paid to CD36 since it promotes uptake of lipoprotein, fatty acid and the like and is expressed in both adipocytes and macrophages, which are the target of AIM function.
3T3-L1 adipocytes were treated with a CD36 neutralizing antibody (Abcam plc; Clone JC63.1 mouse IgA) to inhibit the endocytosis of rAIM. Fig. 22 shows representative photographs of treatment and no treatment with neutralizing antibody, and the number of cells that incorporated rAIM per 100 cells.
As shown in the Figure, the treatment with a CD36 neutralizing antibody markedly suppressed rAIM uptake.

Then, CD36^{+/+} mice (wild-type) and CD36^{-/-} mice (Febbraio et al., J. Biol. Chem., 1999, 274:19055-19062) were intravenously injected with rAIM (300 µg/mouse in PBS), sacrificed 16 hr later, and a section was prepared from adipose tissue. AIM and macrophage F4/80 in the section were stained. The results are shown in Fig. 23.
As shown in the Figure, it was shown that AIM signal markedly decreased in CD36^{-/-} adipocyte, and rAIM uptake capacity was lost in CD36^{-/-} adipocyte.
These results strongly suggest that CD36 causes rAIM uptake.

As mentioned above, AIM is incorporated into adipocytes via CD36 on the cell surface. The level of AIM uptake by adipocytes can be examined easily by, for example, visualizing intracellular AIM using a detectably labeled anti-AIM antibody.
Therefore, using this method, the action mechanism of the candidate compound that suppresses the function of AIM can be clarified, and the effect of said compound can be verified.
For example, when the intracellular uptake of AIM decreases when AIM and a candidate compound that suppresses the function of AIM are added together as compared to the addition of AIM alone, said candidate compound is considered to suppress the function of AIM by inhibiting the intracellular uptake of AIM.

### Reference Example 6. Binding of AIM and FAS

### [Evaluation by immunoprecipitation]

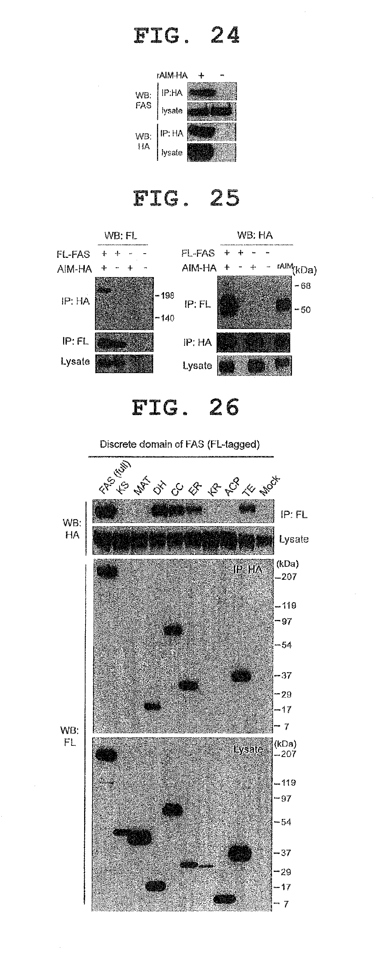

rAIM labeled with HA-tag was directly injected to adipose tissue of AIM^{-/-} mice (at several points, 100 µg in total). After 3 hr, to confirm binding of rAIM-HA and endogenous FAS, co-immunoprecipitation by anti-HA antibody was performed using adipose tissue. The presence of FAS in the precipitate was analyzed by Western blotting (WB).
The results are shown in Fig. 24. As shown in the Figure, it was confirmed that FAS and rAIM were coprecipitated, and incorporated AIM and FAS in the cytoplasm were bound.

In HEK 293T cells, rAIM labeled with HA-tag and FAS labeled with FLAG-tag were co-expressed, and the binding of AIM and FAS was confirmed by co-immunoprecipitation using an anti-Flag antibody or anti-HA antibody.
The results are shown in Fig. 25. rAIM and FAS coprecipitated, and the binding capacity of AIM to FAS was shown.

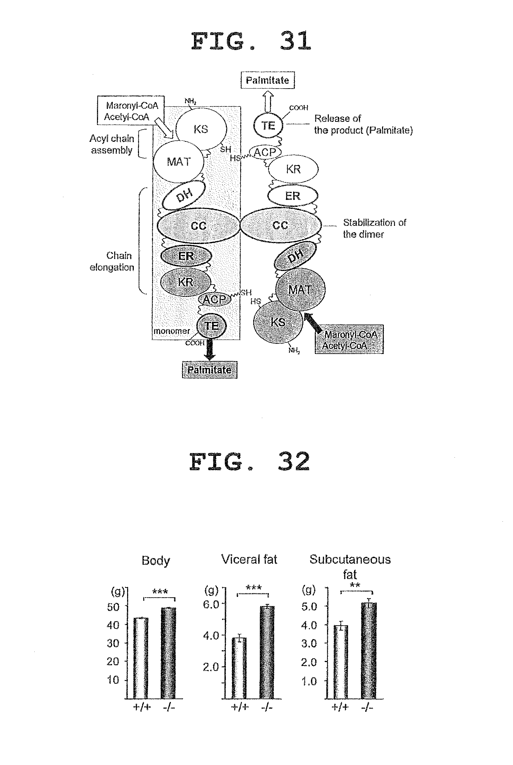

Then, the AIM binding region in FAS was examined. FAS contains 8 domains of keto acyl synthase (KS), malonyl/acetyl transferase (MAT), dehydrase (DH), central core (CC), enoylreductase (ER), keto reductase (KR), acylcarrier protein (ACP) and thioesterase (TE).
The structure of FAS is shown in Fig. 31.
The N-terminal of each domain of FAS was labeled with Flag sequence, and expressed in HEK293T cells where AIM-HA is stably expressed. The binding of AIM-HA and each domain of FAS was confirmed by co-immunoprecipitation using an anti-Flag antibody or anti-HA antibody.
The results are shown in Fig. 26. It was found that the domains bound to AIM were ER, DH, TE and CC.
The sequence encoding full-length FAS cDNA (nucleotide: +1 - 7515) labeled with FLAG-tag was constructed using a part of the cDNA clone provided by Dr. Ohara (Kazusa DNA Research Institute) and some fragments cloned by RT-PCR and pFLAG-CMV2 vector (Sigma).
A cDNA fragment encoding KS, MAT, DH, CC, ER, KR, ACP or TE was prepared by subcloning to pFLAG-CMV2 vector by using full-length FAS cDNA as a template.

### [Suppression of FAS enzyme activity by AIM]

The influence of AIM on FAS activity of adipocyte or adipose tissue was examined. FAS activity was measured by the method of Kelley (Kelley et al., Biochem. J., 1986, 235:87-90) with slight modification. 3T3-L1 adipocytes, or lysates of adipose tissue of obese mice was mixed with acetyl-CoA and NADPH (0.2 M calcium phosphate buffer [pH 7.0] containing 0.4 mM EDTA), and the mixture was placed in a spectrophotometer cuvette and maintained at 30°C. 20 µl of malonyl-CoA solution (0.2 mM) was added to start an enzyme reaction and decrease of OD was measured at a chart speed 2 cm/min. Based on the results, the enzyme activity of FAS was evaluated using the molar extinction coefficient of oxidized NADPH as 6220.

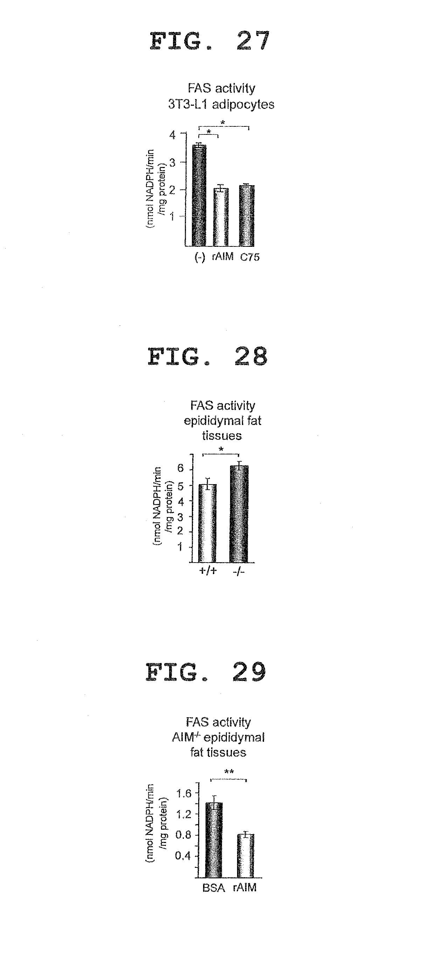

First, the FAS activity of 3T3-L1 cells treated for 6 days in the presence or absence of rAIM (5 µg/ml), and in the presence of C75 (25 µM) was measured. The results are shown in Fig. 27. Moreover, the FAS activity of adipose tissues of AIM^{+/+} mice and AIM^{-/-} mice and the FAS activity of adipose tissue of AIM^{-/-} mice administered with rAIM or BSA 3 hr before by intraadipose topical injection are shown in Figs. 28 and 29, respectively.
All mice were fed with HFD for 20 weeks. The samples were dissolved (lysed) and the FAS activity was measured. The data was converted to that based on FAS protein, according to the results of WB performed using the same sample. In each group, n was 6, and the error bar shows standard error.
As shown in Fig. 27, FAS activity in 3T3-L1 adipocytes was markedly suppressed by the rAIM treatment. The level of suppression was the same as C75 that specifically inhibits FAS.
As compared to AIM^{+/+} mice, adipose tissue significantly increased in vivo in AIM mice (Fig. 28). In addition, direct administration of rAIM of adipose tissue of AIM^{-/-} mice decreased FAS activity.

As mentioned above, AIM binds to FAS and suppresses its activity.
The level of binding of AIM and FAS can be examined easily by immunoprecipitation and the like. Therefore, using this method, the action mechanism of the candidate compound that suppresses the function of AIM can be clarified, and the effect of said compound can be verified. For example, when the binding of AIM and FAS decreases when AIM and a candidate compound that suppresses the function of AIM are added together as compared to the addition of AIM alone, said candidate compound is considered to suppress the function of AIM by inhibiting the binding of AIM and FAS.
The level of suppression of activity can be examined easily by a general method for measuring FAS activity. Therefore, using this method, the action mechanism of the candidate compound that suppresses the function of AIM can be clarified, and the effect of said compound can be verified.
For example, when the suppression of FAS activity is suppressed when AIM and a candidate compound that suppresses the function of AIM are added together as compared to the addition of AIM alone, said candidate compound is considered to suppress the function of AIM by inhibiting the FAS activity suppressive function of AIM.

### Reference Example 7. Changes of body weight and fat amount by AIM (in vivo)

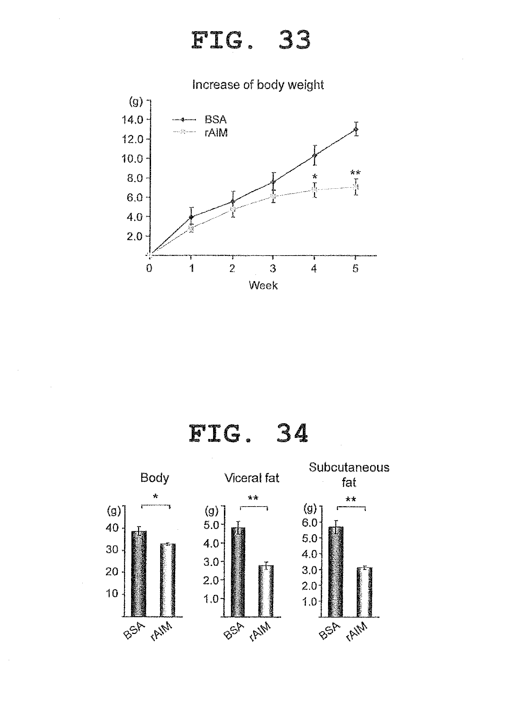

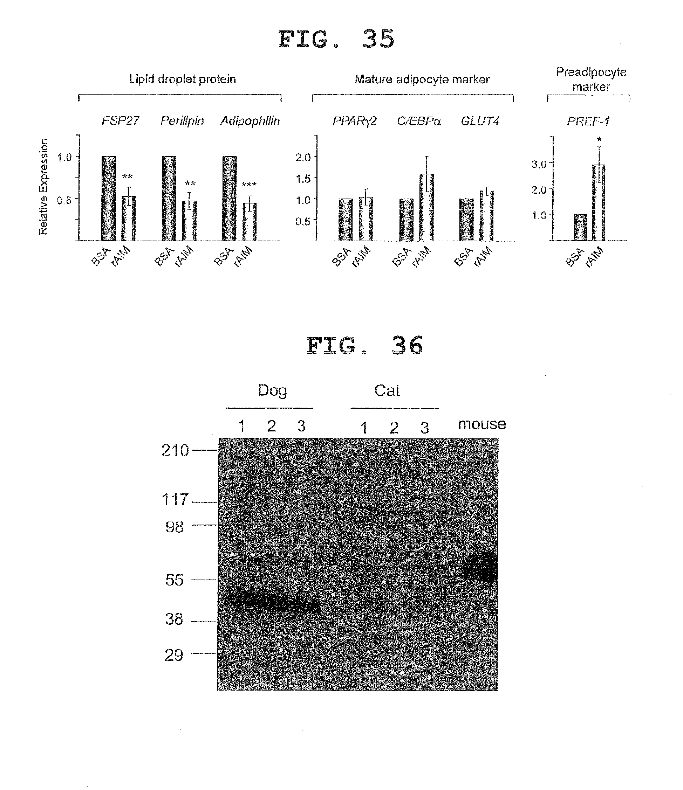

AIM^{+/+} mice (n=7) and AIM^{-/-} mice (n=6) were fed with HFD for 12 weeks, and changes of visceral fat and subcutaneous fat amounts were measured. The results are shown in Fig. 32. The amount of increase in visceral fat (epididymal adipose tissue) and subcutaneous fat was greater in AIM^{-/-} mice.
Then, rAIM was intraperitoneally administered twice a week (n=6; 300 µg/injection/mouse) to AIM^{-/-} mice for 5 weeks while feeding with HFD, and the body weight and adipose tissue amount were measured. The results are shown in Figs. 33 and 34. The rAIM administration group showed a significantly small increase in all of the body weight, visceral fat and subcutaneous fat, as compared to the control administered with bovine serum albumin (n=5; 300 µg/injection/mouse).
The mRNA level of adipocyte marker, preadipocyte marker, and lipid droplet formation-related genes in the RNA extracted from visceral fat tissue of the mice of the rAIM administration group and BSA administration group was measured by QPCR. The results are shown in Fig. 35. The measurement values were standardized with the measurement values of GAPDH, and shown by an expression level relative to that of the adipose tissue administered with BSA.
In the rAIM administration group, the mRNA level of FSP27, Perilipin, and Adipophilin in visceral fat was also low. On the other hand, the mRNA level of PREF-1 increased by the administration of rAIM, but mRNA of PPARγ2, C/EBPα, GLUT4 did not show difference between the rAIM administration group and BSA administration group.

### Reference Example 8. Expression of AIM in dog and cat

AIM protein in the sera of dog, cat and mouse was detected by Western blotting (reducing condition) using an anti-AIM antibody (SA-1).
The results are shown in Fig. 36. As shown in the Figure, AIM protein was detected in all animals. The difference in the molecular weight between species is considered to be attributable to the modification of sugar chain and the like.

### Example 8. AIM inhibitor

An AIM inhibitor was searched by the following screening method.

### <Cell>

3T3-L1 (DS Biopharma Cat No. EC86052701)

### <Reagent>

● medium
   for passage ... DMEM (Invitrogen Cat No. 11995-081)/10% CS/PS SM
   for assay ... DMEM/10% FBS/penicillin + streptomycin for differentiation induction ... DMEM/10% FBS/penicillin + streptomycin/1 µg/mL Insulin/1 µM Dexamethasone/0.5 mM IBMX
● RT-PCR TaqMan Gene Expression Cells-to-Ct kit (Applied Biosystems Cat No. AM1729)
● Probe mouse FSP27, mouse GAPDH (Applied Biosystems Cat No. AM1729)
● mAIM
CHO-S cells under suspension culture using FreeStyle CHO Expression Medium (Invitrogen Cat No. 12651-014) at 37°C were prepared to 10⁶ cells/mL, DMSO was added to the final concentration of 1.25%, and subjected to suspension culture at 37°C for 3 hr. His6-tag was added to the C-terminal of mouse mAIM, and CHO-S cells were transfected with an expression plasmid in which mAIM and His6-tag were connected to the downstream of CMV or PGK promoter. After the cells were cultured at 37°C for 2 hr, temperature was changed to 29°C, culture was continued for 7 days when CMV promoter was used, and 9 days when PGK promoter was used, and the culture supernatant was recovered. The recovered culture supernatant was applied to HisTrap HP column (5 mL, manufactured by GE Healthcare), the column was washed with 20 mM imidazole, and mAIM was eluted with 300 mM imidazole. The recovered solution was substituted with PBS, and concentrated to give purified mAIM.

### <test compound>

- 10 mM test compound in DMSO

### <methods>

### (1) passage of cell

- The cells under culture in Dish were washed with PBS(-), scraped by treating with 1/10 concentration of trypsin, suspended in passage medium and the number of cells was counted.
- Generally, cells were passaged in a 10 cm dish at 5 x10⁵ - 1 x10⁶ cells/dish.
- Cells were seeded in a new 10 cm dish.
   For passage every other day, the cells were seeded at 2 x10⁵ cells/dish, and
   for passage every two days, the cells were seeded at 1 x10⁵ cells/dish.

### (2) Differentiation induction

### - Day 1

- The cells under culture in Dish were washed with PBS(-), scraped by treatment with 1/10 concentration of trypsin, and the number of cells suspended in the assay medium was counted.
- The cells were prepared to 5 x10⁴ cells/mL, and seeded in a 96 well plate (IWAKI) at 100 µL/well (5000 cells/well).

### - Day 3

- A differentiation induction medium added with mAIM (final 400 µg/mL) and diluted test compound (final concentration 10 µM) was prepared.
- The medium of the 96 well plate was removed, and the above-mentioned differentiation induction medium (100 µL/well) was added.

### - Day 5

- The medium was removed, and washed with 100 µL/well of PBS(-) cooled to 4°C.
- 25 µL/well of Lysis Buffer (+1/100 DNase) was added, and the cells were completely lysed by a Plate Shaker (room temperature 1 min).
- The cells were stood still at room temperature for 5 min.
- 2.5 µL/well of Stop Solution was added, and the mixture was mixed by a Plate Shaker (room temperature 2 min).

### (3) RT-PCR assay

The following reverse transcription reaction solution was prepared in a 96 well PCR plate.

| | |
|---|---|
| 2 xRT Buffer | 10 |
| 20 xRT Enzyme Mix | 1 |
| Nuclease-free water | 5 |
| Cell lysate | 4 |
| Total | 20 µL/well |

• reverse transcription reaction
37°C 60 min
95°C 5 min
4°C hold
• The following RT-PCR reaction solution was prepared in a 384 well PCR plate.

| | |
|---|---|
| TaqMan Gene Expression Master Mix | 5 |
| 20x TaqMan Probe (mFSP27) | 0.5 |
| 20x TaqMan Probe (mGAPDH) | 0.5 |
| Nuclease-free water | 2 |
| cDNA sample | 2 |
| Total | 10 µL/well |

• RT-PCR reaction was performed by ABI7900HT.
50°C 2 min
95°C 10 min
40 cycles of the following reaction
95°C 15 sec
60°C 1 min

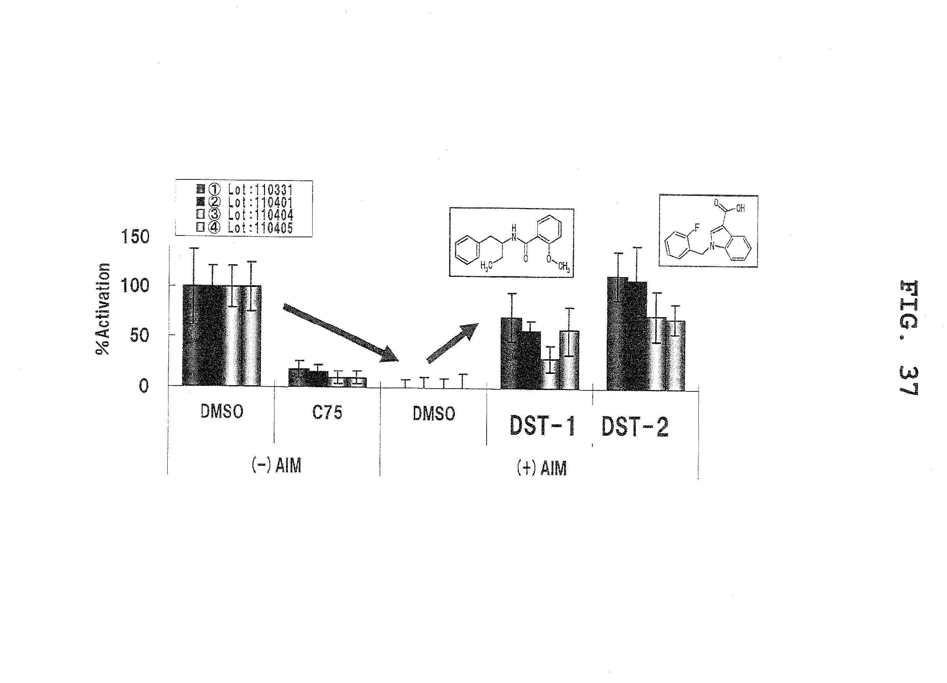

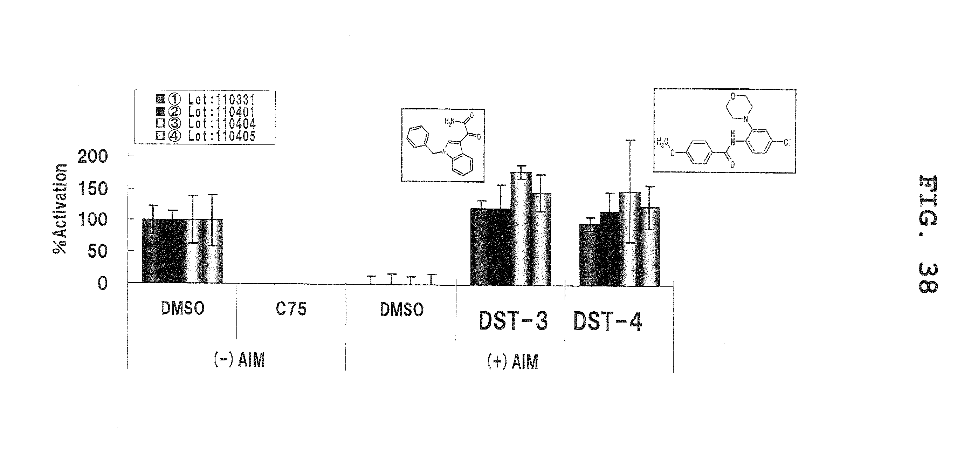

Primary screening was performed with 1,280 compounds (n=1, 10 µm), and the expression level of FSP27 (standardized by GAPD) was measured. The 32 data points (top two data points from each 96 well plate) which showed the activity not more than (-)AIM+3SD hits) were taken as the first hits.
A reproducibility test (n=3, 10 µm, AIM 4 Lot) was performed, and compounds showing activated FSP27 expression level (standardized by GAPD) of not less than (+)AIM+3×SD in all 4 Lots of AIM proteins were selected. The following compounds, DST-1, DST-2, DST-3 and DST-4 in order, were selected. 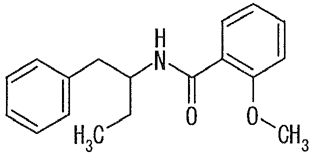 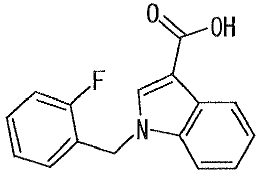 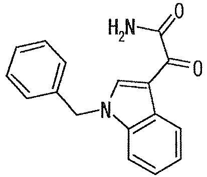 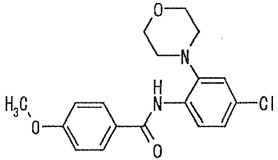 The results of reproducibility tests of DST-1 - DST-4 are shown in Figs. 37, 38. It was confirmed that all compounds significantly suppressed a remarkable decrease of FSP27 mRNA expression induced by the addition of AIM, and inhibited the function of AIM.

### Example 9. Neutralizing antibody

A neutralizing antibody was prepared by the following material and method.

### <animal immunization>

As an antigen, mouse AIM (2 mg/mL) was mixed with an equal amount of TiterMax Gold (G-1 Funakoshi) to give an emulsion. As an animal to be immunized, two 6-week-old female Jcl:Wistar rats (CLEA Japan, Inc.) were used and 50 µL was administered to the hind limb plantar region. After 2 weeks, similar administration was performed, and after 2 weeks or longer, and an antigen solution (50 µg) was administered to the plantar region to prepare for the cell fusion 3 days later.

### <myeloma cell>

As myeloma cell, mouse P3U1 was used and, as a growth culture, a medium prepared by adding glutamine and pyruvic acid to RPMI1640 (11875-119 GIBCO) and adding FBS (S1560 BWT Inc.) to 10% was used. As an antibiotic, an appropriate amount of penicillin or streptomycin was added.

### <cell fusion>

Under anesthesia, heart blood was drawn from a rat, popliteal lymph node was isolated aseptically, placed on a beaker with #200 mesh and a cell suspension was prepared by pushing with a silicon rod. The cells were washed with RPMI1640 by two times of centrifugation, and counted.
Myeloma cells in the logarithmic growth phase were collected by centrifugation, washed, prepared to 5:1 relative to the lymph cell, and subjected to mix centrifugation.
Cell fusion was performed using PEG1500 (783641 Roche). That is, 1 mL of PEG solution was reacted with cell pellets for 3 min, the reaction mixture was stepwisely diluted, and washed by centrifugation. The medium was added, and the mixture was added to 96 well plates (15 plates) by 200 µL, and cultured for 1 week. As the medium, a medium for myeloma cell added with HAT supplement (21060-017 GIBCO) and adjusted to FBS concentration of 15% was used.

### <collection of mouse ascites>

Cryopreserved cells were thawed, growth culture was performed, 1×10⁷ cells were administered to the abdominal cavity of nude mouse (BALB/cAJcl-nu/nu CLEA Japan, Inc.) intraperitoneally administered with 0.5 ml of pristine (42-002 COSMO BIO) not less than 1 week before, and 4 - 12 ml of ascites was obtained about 2 weeks later. Solid was removed by centrifugation treatment and the ascites was cryopreserved.

### <Electrophoresis analysis>

The ascites was thawed, treated with 5 µm filter, and the band of a monoclonal antibody contained in the ascites was confirmed by cellulose acetate membrane electrophoresis.
The electrophoresis conditions were 0.05 M Barbital Na Buffer pH 8.6 (020-13415 Wako Pure Chemical Industries, Ltd.), SELECA-V (ADVANTEC), 1 mA/cm, 25 min, fixing was performed, and 0.1% nigrosine (2% acetic acid) was used for staining.

### <Antibody assay>

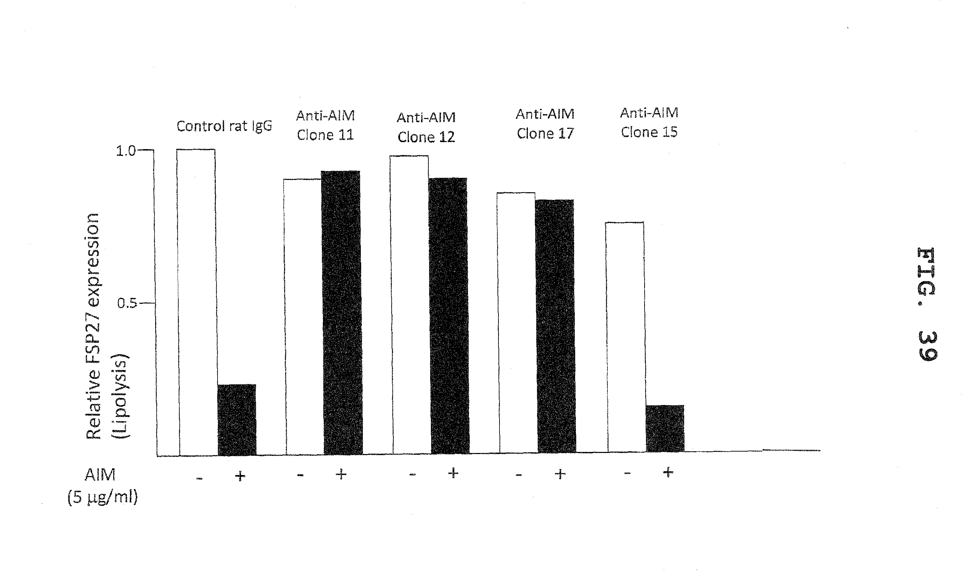

24 anti-mouse AIM rat monoclonal antibodies, each at a concentration of 200 mg/mL, were treated with rAIM at room temperature for 30 min and added to differentiated 3T3-L1 adipocytes (day 4 of differentiation induction) at rAIM final concentration of 5 mg/mL, and cultured for 24 hr. RNA was isolated from the cells, and the mRNA amount of FSP27 was quantified by quantitative RT-PCR.
The results are shown in Fig. 39.
In 3 clones of clones 11, 12 and 17, a remarkable decrease of FSP27 mRNA expression inherently induced by the addition of rAIM was significantly suppressed. It was not suppressed in the control rat IgG and treatment with clone 15.

### Example 10. Measurement of blood hAIM concentration of subjects who underwent medical checkup

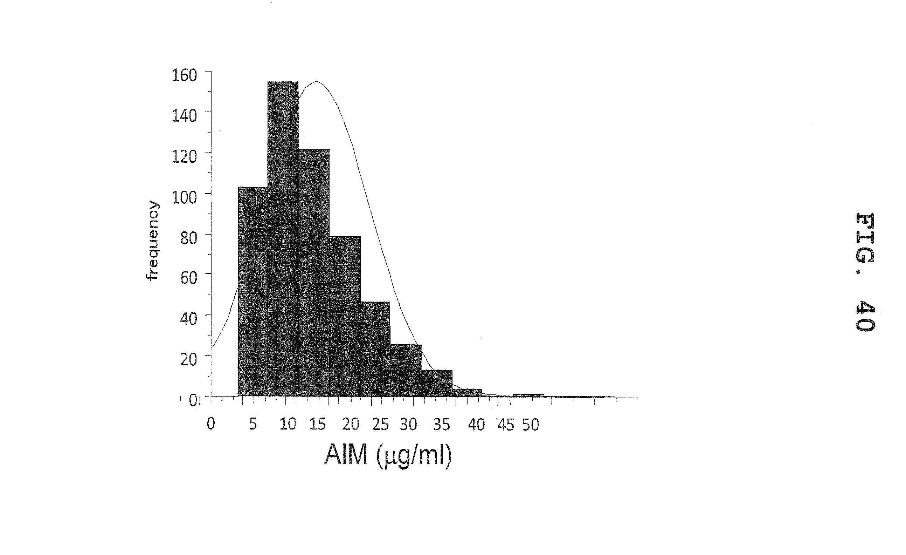

In the same manner as in Example 9 except that human rAIM was used as antigen, an anti-AIM antibody was obtained.
Of the obtained clones, AIM-CL-6 and AIM-CL-7 were deposited according to the prescription of the Budapest Treaty. The deposit Nos. were NITE BP-1092 and NITE BP-1093, respectively.
Depository: Incorporated Administrative Agency National Institute of Technology and Evaluation Patent Microorganisms Depositary (NPMD: National Institute of Technology and Evaluation, Patent Microorganisms Depositary; 2-5-8 Kazusakamatari, Kisarazu-shi, Chiba, Japan)
Deposit date: May 2, 2011
Using AIM-CL-6 and AIM-CL-7, the blood hAIM concentration of about 550 subjects who underwent medical checkups was measured. To be specific, using AIM-CL-6 for trapping, AIM-CL-7 for detection, and serum (50 µL), the analysis was performed in duplicate. Human rAIM was sequentially diluted to set a standerd curve, and the concentration was determined thereby.
Human rAIM was obtained by producing human AIM protein labeled with HA-tag in HEK293T cells, and column purifying the protein from the culture supernatant using an anti-HA antibody.
The results are shown in Fig. 40.

### Example 11. Relationship between BMI and blood AIM concentration

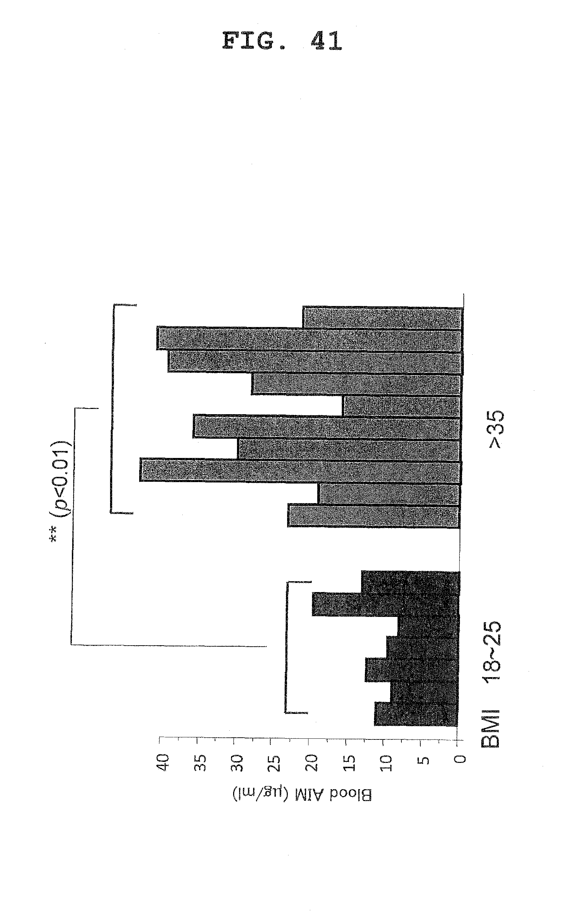

From blood donors (including foreigners), donors having BMI of 18 - 25 or not less than 35 were selected at random, and the blood AIM concentration was measured by a method similar to that in Fig. 6. Generally, a higher BMI is said to be associated with a higher risk of metabolic syndrome.
The results are shown in Fig. 41. The blood AIM concentration was significantly high in those having BMI of not less than 35, as compared to those having BMI of 18 - 25. This strongly suggests that blood AIM concentration correlates with the risk of metabolic syndrome, and inhibition of AIM enables prophylaxis or treatment of a metabolic syndrome.

### [Sequence Listing Free Text]

SEQ ID NO: 1 is the amino acid sequence of human AIM.
SEQ ID NO: 2 is the DNA sequence of forward primer used for the PPARγ1 expression analysis by quantitative real-time PCR.
SEQ ID NO: 3 is the DNA sequence of reverse primer used for the PPARγ1 expression analysis by quantitative real-time PCR.
SEQ ID NO: 4 is the DNA sequence of forward primer used for the PPARγ2 expression analysis by quantitative real-time PCR.
SEQ ID NO: 5 is the DNA sequence of reverse primer used for the PPARγ2 expression analysis by quantitative real-time PCR.
SEQ ID NO: 6 is the DNA sequence of forward primer used for the C/EBPα expression analysis by quantitative real-time PCR.
SEQ ID NO: 7 is the DNA sequence of reverse primer used for the C/EBPα expression analysis by quantitative real-time PCR.
SEQ ID NO: 8 is the DNA sequence of forward primer used for the CD36 expression analysis by quantitative real-time PCR.
SEQ ID NO: 9 is the DNA sequence of reverse primer used for the CD36 expression analysis by quantitative real-time PCR.
SEQ ID NO: 10 is the DNA sequence of forward primer used for the GLUT4 expression analysis by quantitative real-time PCR.
SEQ ID NO: 11 is the DNA sequence of reverse primer used for the GLUT4 expression analysis by quantitative real-time PCR.
SEQ ID NO: 12 is the DNA sequence of forward primer used for the Fsp27 expression analysis by quantitative real-time PCR.
SEQ ID NO: 13 is the DNA sequence of reverse primer used for the Fsp27 expression analysis by quantitative real-time PCR.
SEQ ID NO: 14 is the DNA sequence of forward primer used for the Perilipin expression analysis by quantitative real-time PCR.
SEQ ID NO: 15 is the DNA sequence of reverse primer used for the Perilipin expression analysis by quantitative real-time PCR.
SEQ ID NO: 16 is the DNA sequence of forward primer used for the Adipophilin expression analysis by quantitative real-time PCR.
SEQ ID NO: 17 is the DNA sequence of reverse primer used for the Adipophilin expression analysis by quantitative real-time PCR.
SEQ ID NO: 18 is the DNA sequence of forward primer used for the GAPDH expression analysis by quantitative real-time PCR.
SEQ ID NO: 19 is the DNA sequence of reverse primer used for the GAPDH expression analysis by quantitative real-time PCR,
SEQ ID NO: 20 is the DNA sequence of forward primer used for the PREF1 expression analysis by quantitative real-time PCR.
SEQ ID NO: 21 is the DNA sequence of reverse primer used for the PREF1 expression analysis by quantitative real-time PCR.
SEQ ID NO: 22 is the amino acid sequence of chimpanzee AIM.
SEQ ID NO: 23 is the amino acid sequence of dog AIM.
SEQ ID NO: 24 is the amino acid sequence of mouse AIM.
SEQ ID NO: 25 is the amino acid sequence of rat AIM.

## Claims

1. A method for the prophylaxis or treatment of metabolic syndrome or related diseases thereof, comprising a step of administering an AIM inhibitor to a subject.

2. The method according to claim 1, wherein the aforementioned AIM inhibitor decreases stability of AIM in blood.

3. The method according to claim 1, wherein the aforementioned AIM inhibitor inhibits binding between AIM and CD36.

4. The method according to claim 1, wherein the aforementioned AIM inhibitor inhibits incorporation of AIM into a target cell.

5. The method according to claim 1, wherein the aforementioned AIM inhibitor inhibits transfer of AIM from endosome to cytoplasm.

6. The method according to claim 1, wherein the aforementioned AIM inhibitor inhibits binding of AIM to fatty acid synthase (FAS).

7. The method according to claim 1, wherein the aforementioned AIM inhibitor suppresses an expression of AIM.

8. The method according to any one of claims 1 to 7, wherein the aforementioned metabolic syndrome or related diseases thereof is at least one selected from the group consisting of metabolic syndrome, obesity, insulin resistance, diabetes, hyperlipidemia, hypertension, arteriosclerotic disease, hepatic disease, liver dysfunction, cerebrovascular disorder, ischemic heart disease, heart failure, dementia, cerebral apoplexy, neurosis, renal disease, secretion abnormality of adipocytokine, and abnormal amount of blood free fatty acid.

9. The method according to any one of claims 1 to 8, wherein the aforementioned subject is a human.

10. The method according to any one of claims 1 to 8, wherein the aforementioned subject is a non-human mammal or bird.

11. The method according to claim 10, wherein the aforementioned subject is a dog or cat.

12. An agent for the prophylaxis or treatment of metabolic syndrome or related diseases thereof, comprising at least one selected from the following group:
an anti-AIM antibody;
an anti-CD36 antibody;
a double stranded nucleic acid having RNAi effect for an AIM gene;
an antisense nucleic acid to an AIM gene;
a ribozyme to an AIM gene;
a protein binding to an anti-AIM antibody and not suppressing FAS activity;
a protein binding to CD36 and not suppressing FAS activity;
a protein binding to FAS and not suppressing FAS activity; and
a soluble CD36.

13. The therapeutic agent according to claim 12, wherein the aforementioned antibody is a monoclonal antibody, a polyclonal antibody, a recombinant antibody, a human antibody, a humanized antibody, a chimeric antibody, or an antibody fragment.

14. The therapeutic agent according to claim 12 or 13, wherein the aforementioned protein binding to FAS and not suppressing FAS activity binds to at least one domain selected from the group consisting of DH, ER, TE and CC.

15. The therapeutic agent according to claim 12 or 13, wherein the aforementioned protein binding to an anti-AIM antibody and not suppressing FAS activity is selected from the group consisting of an AIM fragment, an AIM variant or a fragment thereof, and an AIM chimeric protein or a fragment thereof.

16. The therapeutic agent according to claim 12 or 13, wherein the aforementioned protein binding to CD36 and not suppressing FAS activity is selected from the group consisting of an AIM fragment, an AIM variant and an AIM chimeric protein.

17. The therapeutic agent according to claim 12 or 13, wherein the aforementioned protein binding to FAS and not suppressing FAS activity is selected from the group consisting of an AIM fragment, an AIM analog, an AIM variant and an AIM chimeric protein.

18. The therapeutic agent according to any one of claims 15 to 17, wherein the aforementioned AIM fragment is selected from fragments containing a functional domain of AIM protein and a conserved region thereof.

19. The therapeutic agent according to any one of claims 12 to 18, wherein the aforementioned metabolic syndrome or related diseases thereof is at least one selected from the group consisting of metabolic syndrome, obesity, insulin resistance, diabetes, hyperlipidemia, hypertension, arteriosclerotic disease, hepatic disease, liver dysfunction, cerebrovascular disorder, ischemic heart disease, heart failure, dementia, cerebral apoplexy, neurosis, renal disease, secretion abnormality of adipocytokine, and abnormal amount of blood free fatty acid.

20. The therapeutic agent according to any one of claims 12 to 19, wherein the aforementioned subject is a human.

21. The therapeutic agent according to any one of claims 12 to 19, wherein the aforementioned subject is a non-human mammal or bird.

22. The therapeutic agent according to claim 21, wherein the aforementioned subject is a dog or cat.

23. A method for the prophylaxis or treatment of metabolic syndrome or related diseases thereof, comprising a step of administering the therapeutic agent according to any one of claims 12 to 22.

24. A method of producing an AIM inhibitor, comprising
a step of treating AIM with a protease to give an AIM fragment, and
a step of purifying the aforementioned AIM fragment by an affinity column wherein an anti-AIM antibody binding to an AIM functional domain or conserved region is immobilized.
